# EUROPÄISCHE PATENTSCHRIFT

(11) **EP 3 673 264 B1**
(45) Veröffentlichungstag und Bekanntmachung des Hinweises auf die Patenterteilung: **19.10.2022**
(21) Anmeldenummer: 18762478.8
(22) Anmeldetag: 24.08.2018
(51) Int. Cl.: G01N 33/14, G01N 21/3504, G01N 21/84

(54) **VORRICHTUNG UND ZUGEHÖRIGES VERFAHREN ZUR UNTERSUCHUNG BEI LEBENSMITTELN IN VERPACKUNGEN**
DEVICE AND CORRESPONDING ANALYSIS METHOD FOR FOODSTUFF IN PACKAGING
DISPOSITIF ET PROCÉDÉ CORRESPONDANT D'ANALYSE DE NOURRITURE DANS DES EMBALLAGES

(30) Priorität: 24.08.2017 DE 102017119380
(43) Veröffentlichungstag der Anmeldung: 01.07.2020
(73) Patentinhaber: Steinfurth Mess-Systeme GmbH, 45309 Essen (DE)
(72) Erfinder: ANGRES, Johann, 44803 Bochum (DE); FALKENSTEIN, Martin, 44795 Bochum (DE)
(74) Vertreter: Bals & Vogel Patentanwälte PartGmbB
(86) Internationale Anmeldenummer: PCT/EP2018/072935
(87) Internationale Veröffentlichungsnummer: WO 2019/038444

(56) Entgegenhaltungen:
- WO-A1-2010/145892
- WO-A1-2010/145892
- WO-A1-2010/145892
- WO-A1-2015/036399
- WO-A1-2015/036399
- WO-A1-2015/055743
- WO-A1-2015/055743
- WO-A1-2016/001672
- WO-A1-2016/001672
- WO-A1-2016/001672
- DE-A1-102013 008 003
- DE-A1-102013 008 003
- DE-A1-102013 008 003
- RO-B1- 128 431
- RO-B1- 128 431
- RO-B1- 128 431
- US-B1- 9 316 554
- US-B1- 9 316 554
- US-B1- 9 316 554

## Beschreibung

Die vorliegende Erfindung betrifft ein Prüfgerät gemäß der im Oberbegriff des unabhängigen Vorrichtungsanspruchs näher definierten Art. Ferner bezieht sich die Erfindung auf ein System gemäß dem Oberbegriff des unabhängigen Systemanspruchs sowie ein Verfahren gemäß dem Oberbegriff des unabhängigen Verfahrensanspruchs.

Aus dem Stand der Technik sind diverse Verfahren zur Untersuchung von Lebensmitteln in Verpackungen bekannt. Derartige Verfahren betreffen z. B. jede Form von Getränken sowie Milchprodukte, in Form von Käse, Joghurt sowie Fleischwaren, Süßigkeiten, Gewürze und dergleichen. Aus der DE 10 2013 008 003 A1 und der RO 128 431 A2 ist beispielsweise eine Untersuchung eines Oberflächenbelags bzw. von Oberflächeneigenschaften auf einem Lebensmittel, insbesondere auf Fleisch, bekannt. Ferner bekannt sind bspw. Haltbarkeitsuntersuchungen. So geben Lebensmittelhersteller das Mindesthaltbarkeitsdatum (kurz MHD genannt) zu ihren Lebensmitteln an, bis zu welchem Termin das Lebensmittel bei sachgerechter Aufbewahrung (insbesondere Einhaltung der im Zusammenhang mit dem Mindesthaltbarkeitsdatum genannten Lagertemperatur) auf jeden Fall ohne wesentliche Geschmacks- und Qualitätseinbußen sowie gesundheitliches Risiko zu konsumieren ist. Bei dem Mindesthaltbarkeitsdatum handelt es sich nicht um ein Verfallsdatum, da i. d. R. die Lebensmittel auch nach dem angegebenen Mindesthaltbarkeitsdatum, noch verzehrfähig sind.

Die Lebensmittelhersteller verwahren von jeder Herstellungsserie von Lebensmittel Proben auf, anhand derer sie die Qualität der Serie von Lebensmittelprodukten nachweisen können. Außerdem werden hier die einzelnen Proben von Lebensmitteln aus einer Serie zu vorgegebenen Zeitpunkten auf ihre Qualität hin überprüft. Die bereits untersuchten Proben von Lebensmitteln sind jedoch i. d. R. nach der Untersuchung unbrauchbar, da die Verpackungen geöffnet worden sind und somit Luft an die Lebensmittel gelangen konnte. Die US 9,316,554 B1 schlägt beispielsweise ein faseroptisches analytisches Messgerät vor, welches in die Lebensmittelverpackung eingestochen wird. Gemäß der Lehre der WO 2016/ 001672 A1 wird eine Flasche geöffnet, um mit Hilfe einer Brennstoffzelle den Alkoholgehalt zu bestimmen. Außerdem wird i. d. R. durch die Untersuchung auch die Aufbewahrungstemperatur verändert, was ebenfalls nicht gewünscht ist und sich auf das Lebensmittel negativ auswirken kann. Bei der WO 2010/145892 A1 wird zur Erfassung der Lichtstreuung beispielsweise der Deckel von der zu untersuchenden Flasche abgenommen, um den Detektor an dieser Stelle zu platzieren. Aus diesem Grund muss der Lebensmittelhersteller entsprechend viele Proben von Lebensmitteln aufbewahren, um zu den jeweiligen Zeitpunkten eine zerstörende Probemessung vornehmen zu können. Hierdurch ist es notwendig, dass der Lebensmittelhersteller entsprechend viele Proben aus einer Serie von Lebensmitteln für spätere Untersuchungen bevorraten muss. Somit ist der Aufbewahrungs- und Bevorratungsbedarf sowie die dadurch entstehenden Verluste bei der Lebensmittelproduktion sehr groß.

Ferner sind Untersuchungen bei Lebensmitteln in Verpackungen nach dem Stand der Technik sehr aufwendig und unflexibel. Meist umfassen solche Untersuchungen mehrere im Labor durchzuführende Schritte, bei welchen die Probe umfangreich vorbereitet und mittels komplexer stationärer Vorrichtungen analysiert werden muss. Ein Ziel solcher Untersuchungen ist bspw. die Erfassung eines CO₂-Gehalts von flüssigen Lebensmitteln, welche mit Kohlensäure versetzt sind.

Aus der WO 2015/055743 A1 sind ein System und ein Verfahren zur Berechnung von Kohlendioxid im Kopfraum von einem verschlossenen Behälter gefüllt mit einer Flüssigkeit bekannt, wobei mit Hilfe einer LED und eines optischen Filters unterschiedliche Wellenlängen auf und abseits einer Absorptionswellenlänge von CO₂ gemessen werden.

Bei solchen Lebensmitteln in Form von Getränken hat sich herausgestellt, dass der vorhandene Druck in der Flasche sowie das Material der Flasche, beispielsweise Glas, Porzellan oder PET maßgeblich für die Qualität und den Geschmack der Getränke ist. Damit kommt auch der Erfassung des Drucks durch entsprechende Untersuchungen eine besondere Bedeutung zu. Auch hier wird das Nutzen dieser Untersuchungen oft durch die Flexibilität und den (Kosten-) Aufwand eingeschränkt.

Aufgabe der vorliegenden Erfindung ist es somit, die zuvor beschriebenen Nachteile aus dem Stand der Technik zumindest teilweise zu überwinden. Insbesondere ist es Aufgabe der vorliegenden Erfindung, eine verbesserte Möglichkeit zur Untersuchung von Lebensmitteln bereitzustellen. Hierbei soll insbesondere die Verpackung der Lebensmittel bei der Untersuchung unbeeinträchtigt bzw. voll funktionsfähig bleiben, um die bestmögliche Haltbarkeit des Lebensmittels zu gewährleisten.

Die voranstehende Aufgabe wird gelöst durch ein Prüfgerät mit den Merkmalen des unabhängigen Vorrichtungsanspruchs, ein System mit den Merkmalen des Systemanspruchs sowie durch ein Verfahren mit den Merkmalen des unabhängigen Verfahrensanspruchs. Weitere Merkmale und Details der Erfindung ergeben sich aus den jeweiligen Unteransprüchen, der Beschreibung und den Zeichnungen. Dabei gelten Merkmale und Details, die im Zusammenhang mit dem erfindungsgemäßen Prüfgerät beschrieben sind, selbstverständlich auch im Zusammenhang mit dem erfindungsgemäßen System sowie dem erfindungsgemäßen Verfahren, und jeweils umgekehrt, so dass bezüglich der Offenbarung zu den einzelnen Erfindungsaspekten stets wechselseitig Bezug genommen wird bzw. werden kann.

Die Aufgabe wird gelöst durch ein Prüfgerät nach Anspruch 1 zur zerstörungsfreien Erfassung der Gas-Konzentration, vorzugsweise zur CO₂-Erfassung, bei Lebensmitteln in Verpackungen, aufweisend:
- eine Erfassungsanordnung zur Erfassung wenigstens eines Parameters bei einem Lebensmittel in einer Verpackung, wobei der Parameter für eine zu untersuchende Eigenschaft des Lebensmittels spezifisch ist.

Hierbei ist es vorgesehen, dass das Prüfgerät als ein Handheld-Gerät ausgebildet ist. Somit ist eine besonders flexible und kostengünstige Erfassung möglich, insbesondere mobil ohne die Nutzung stationärer Erfassungsgeräte. Es kann hierbei die Erfassung des zumindest einen Parameters als eine Erfassung, insbesondere eine Messung, von Druck und/oder Temperatur bei dem Lebensmittel zur Bestimmung eines CO₂-Gehalts bei dem Lebensmittel und/oder in der Verpackung ausgeführt sein. In anderen Worten sind hierbei die Parameter Druck und/oder Temperatur für den CO₂-Gehalt spezifisch, d. h. der CO₂-Gehalt kann anhand dieser Parameter (zumindest näherungsweise) bestimmt werden. Ferner können alternativ oder zusätzlich zum CO₂-Gehalt auch weitere zu untersuchende Eigenschaften des Lebensmittels von Interesse sein. Hierbei kann es sich um physikalische Eigenschaften oder auch um Qualitätseigenschaften des Lebensmittels handeln. Bspw. kann der erfasste Parameter auch direkt die Eigenschaft bilden, welche untersucht werden soll (dies ist z. B. der Fall, wenn der Druck oder die Temperatur des Lebensmittels als ein entsprechender Parameter untersucht wird). Es können im Rahmen der Erfindung gleichwohl auch mehrere Parameter erfasst werden, um eine (einzige) Eigenschaft des Lebensmittels zu bestimmen (So können z. B. Druck und Temperatur miteinander verglichen und/oder kombiniert ausgewertet werden, um einen CO₂-Gehalt zu erhalten.). Ein besonderer Vorteil ergibt sich dadurch, dass das erfindungsgemäße Prüfgerät vollständig als Handheld-Gerät ausgeführt ist. Damit ist eine solche - üblicherweise durch stationäre Geräte durchgeführte - Untersuchung nunmehr einfach und kostengünstig durchführbar.

Vorteilhafterweise wird unter einem Handheld (Handheld-Gerät) ein tragbares elektronisches Gerät verstanden, bei welchem vorzugsweise eine mobile Energieversorgung vorgesehen ist, z. B. durch wenigstens einen (ggf. festen oder wechselbaren) wiederaufladbaren Akkumulator und/oder wenigstens eine (ggf. feste oder wechselbare) nicht-wiederaufladbare Batterie. Dabei kann der Handheld als ein Handgerät dazu ausgebildet sein, bei der Benutzung in nur einer Hand gehalten zu werden, sodass das Handheld-Gerät durch eine Hand tragbar und/oder bedienbar ausgeführt sein kann.

Es werden im Folgenden die Begriffe "Lebensmittel" und "Verpackungen" erwähnt, womit insbesondere gemeint ist, dass es sich um die gleichen oder gleichartigen Lebensmittel handelt. Auch können diese Lebensmittel in den gleichen oder gleichartigen Verpackungen angeordnet sein. Unter dem Begriff "Probe" wird das zu untersuchende Lebensmittel in der Verpackung verstanden, und als "Produkt" wird die Kombination aus Lebensmittel und Verpackung verstanden.

Die zu untersuchende Eigenschaft, insbesondere der CO₂-Gehalt eines Lebensmittels, kann z. B. anhand einer Vorgabe, wie einer Berechnungsformel oder eines Gesetzes, bestimmt werden. So beschreibt das Henry-Dalton-Gesetz die Löslichkeit von Gasen in einer Flüssigkeit. Entsprechend können hierbei Parameter wie der Partialdruck (oder auch in Näherung der Gesamtdruck) des Gases sowie die Konzentration des Gases im Lösungsmittel (bspw. einer Flüssigkeit des Lebensmittels) genutzt werden, um die zu untersuchende Eigenschaft zu bestimmen. Auch ist es denkbar, dass Randbedingungen zur Bestimmung der zu untersuchenden Eigenschaft festgelegt werden müssen. Hierzu können weitere Parameter erfasst werden, wie z. B. eine Temperatur des Lebensmittels.

Des Weiteren kann vorgesehen sein, dass die zu untersuchende Eigenschaft ein CO₂-Gehalt des Lebensmittels in der Verpackung ist. Dies ermöglicht es, eine Qualität des Lebensmittels zuverlässig zu beurteilen. Insbesondere kann der CO₂-Gehalt dazu genutzt werden, die Haltbarkeit des Lebensmittels zu prüfen. Die tragbare Ausführung des Prüfgeräts als Handheld ist hierbei von großem Vorteil, da die Untersuchung dann nicht nur aufwendig im Labor, sondern direkt beim Verkauf oder auch durch den Kunden erfolgen kann.

Ferner kann es im Rahmen der Erfindung vorgesehen sein, dass der Parameter ein Druck und/oder eine Temperatur bei dem Lebensmittel ist. Der Druck kann dabei bspw. ein Gesamtdruck und/oder ein Partialdruck bei dem Lebensmittel in der Verpackung sein, um ggf. anhand der Temperatur zuverlässig die zu untersuchende Eigenschaft zu bestimmen. Bspw. wird die Erfassung der Temperatur bei dem Lebensmittel separat von der Erfassung des Drucks durchgeführt, also durch separate Sensoren, wobei die Erfassungen durch die Sensoren auch gleichzeitig durchgeführt werden können.

Es können auch verschiedene Drücke und/oder verschiedene physikalische Eigenschaften als Parameter erfasst werden, und/oder aus einem der erfassten Parameter (z. B. aus dem Gesamtdruck) der weitere Parameter (wie der Partialdruck) berechnet werden. Bspw. kann lediglich der Gesamtdruck in einfacher Weise bei dem Lebensmittel in der Verpackung erfasst werden, jedoch ist der Partialdruck ggf. zur Bestimmung der zu untersuchenden Eigenschaft notwendig (insbesondere gemäß dem Henry-Dalton-Gesetz zur Bestimmung des CO₂-Gehalts). Es kann zur Lösung dieses Problems somit eine weitere Information notwendig sein, d. h. wenigstens eine Kalibrierungsinformation, um aus dem Gesamtdruck den Partialdruck zu bestimmen und/oder um die zu untersuchende Eigenschaft nur anhand des erfassten Parameters zu bestimmen.

Von Vorteil ist es daher im Rahmen der Erfindung, wenn zumindest eine Kalibrierungsinformation für die Durchführung der Erfassung und/oder für die Bestimmung der zu untersuchenden Eigenschaft genutzt wird, insbesondere ausgewählt wird. Bevorzugt erfolgt die Auswahl aus mehreren vorgespeicherten Kalibrierungsinformationen, z. B. aus einer Datenbank. Es kann durch die Kalibrierungsinformation angegeben werden, wie bei einem bestimmten Lebensmittel und/oder bei einer bestimmten Verpackung das Ergebnis (z. B. der CO₂-Gehalt) und/oder der andere Parameter (z. B. Partialdruck) aus dem erfassten Parameter (z. B. Gesamtdruck) berechnet wird. Hierzu wird bspw. durch die Kalibrierungsinformation eine mathematische Formel vorgegeben und/oder ein Algorithmus parametrisiert werden.

Zur Auswahl der richtigen Kalibrierungsinformation muss ggf. eine Zusatzinformation, z. B. über die Art des Lebensmittels und/oder über die Verpackung, bekannt sein. Eine solche Zusatzinformation zur Auswahl der Kalibrierungsinformation kann bspw. eine Information über das Lebensmittel in der Verpackung und/oder über die Verpackung (produktspezifische Information) sein, bspw. eine Bildinformation (wie ein Foto des Lebensmittels in der Verpackung), eine manuelle Auswahl des Lebensmittels und/oder der Verpackung, eine Information über eine Geometrie der Verpackung, eine messtechnische Erfassung der Verpackung oder dergleichen. Diese Zusatzinformation kann z. B. in einem separaten Schritt durch ein externes Gerät erfasst werden. Sodann kann anhand der Zusatzinformation die wenigstens eine Kalibrierungsinformation ausgewählt werden, welche im Anschluss zur Bestimmung der Eigenschaft und/oder des anderen Parameters aus dem erfassten Parameter dient.

Erfindungsgemäß weist die Erfassungsanordnung zumindest eine Sendeeinheit zur Aussendung einer Messeinwirkung und zumindest eine Empfangseinheit zur Erfassung der ausgesendeten Messeinwirkung auf. Erfindungsgemäß ist die Sendeeinheit als eine Strahlungsquelle in Form einer Laserquelle, und die Empfangseinheit als ein optischer Sensor und die Messeinwirkung als eine Strahlung in Form einer Laserstrahlung ausgeführt. Die Erfassungsanordnung ist dazu ausgeführt, eine Veränderung eines Spektrums der Laserstrahlung zu erfassen, welche für den zu erfassenden Parameter spezifisch ist. Bspw. weist der optische Sensor wenigstens eine Photodiode auf, sodass bei einem Verändern der Wellenlänge der Laserstrahlung während der Erfassung ein Spektrum ermittelt werden kann. Dies ermöglicht es, einfach und kostengünstig die Erfassung durchzuführen. Ferner kann bei Nutzung eines kompakten Lasers, wie eines Diodenlasers, als Strahlungsquelle die Erfassung auch besonders flexibel und mobil durchgeführt werden. Es wird damit der Vorteil erzielt, dass durch ein Erfassungsergebnis, wie dem Spektrum, zuverlässig der zu erfassende Parameter bestimmt werden kann. Dies kann bspw. durch eine Auswertung von Peaks oder dergleichen im Spektrum erfolgen, wobei ggf. eine Breite (z. B. Halbwertsbreite) dieser Peaks für den Parameter spezifisch ist.

Es ist denkbar, dass die Empfangseinheit der Erfassungsanordnung als wenigstens ein Sensor ausgebildet ist, welcher wenigstens eine Photodiode oder dergleichen aufweist. Die wenigstens eine Sendeeinheit ist bspw. als eine Lichtquelle (Strahlungsquelle) ausgebildet, welche vorzugsweise einen Halbleiterlaser, insbesondere einen durchstimmbaren Halbleiterlaser, aufweist. Dieser kann z. B. wenigstens eine Laserdiode aufweisen, um die Strahlung zu emittieren. Die Lichtquelle wird z. B. mit einem elektrischen Strom veränderlicher Stromstärke betrieben. Dabei kann die Lichtquelle dazu ausgeführt sein, anhand der Stromstärke durchgestimmt zu werden. Die Wellenlänge der durch die Lichtquelle emittierten Strahlung ist somit strom- und ggf. temperaturabhängig, wobei die mittlere (Center-) Wellenlänge im Bereich von 500 bis 4000 nm, vorzugsweise 1500 bis 2500 nm liegen kann. Dies ermöglicht es, zuverlässig ein angemessen breites Spektrum durch die Erfassungsanordnung zu erfassen. Zur Kühlung kann die Laserdiode der Lichtquelle bspw. an einem Kühlelement angeordnet sein, z. B. an einem Peltier-Element.

Optional kann das Prüfgerät und/oder die Erfassungsanordnung auch wenigstens ein Mittel zur Erfassung einer Dichte und/oder eines Sauerstoffgehalts (jeweils z. B. des Lebensmittels) und/oder eines Drehmoments bei der Verpackung, insbesondere eines Verschlusses der Verpackung, aufweisen. Diese Erfassung kann mit der Erfassung des Drucks und/oder der Erfassung des CO₂-Gehalts kombiniert werden, um eine Zuverlässigkeit der Untersuchung zu erhöhen.

Erfindungsgemäß ist ferner eine Positionierungsstruktur vorgesehen, um eine äußere Positionierung (d. h. eine Positionierung von außen) der Erfassungsanordnung an der Verpackung durchzuführen, insbesondere gemäß einer vorbestimmten Anordnung, insbesondere mit vorbestimmten Abständen und/oder zentriert zur Verpackung. Hierbei ist der Hintergrund, dass eine exakte Positionierung mit vorgegebenen Abständen und Winkeln zur Verpackungsgeometrie notwendig sein kann, um die Erfassung korrekt auszuführen. Insbesondere wird bei der Erfassung wenigstens eine Kalibrierungsinformation (ggf. mit Informationen über die Verpackungsgeometrie) genutzt, um den wenigstens einen Parameter und/oder die Eigenschaft des Lebensmittels zuverlässig zu bestimmen. Da diese Kalibrierungsinformation ggf. von der exakten geometrischen Ausrichtung abhängig ist, muss diese durch die Positionierungsstruktur gewährleistet werden. Hierzu sind bspw. die Sensoren, wie eine Sende- und/oder Empfangseinheit und/oder ein Temperatursensor, über ein Gehäuse und/oder einen Rahmen am Prüfgerät fest und in definierter Weise befestigt.

In einer weiteren Möglichkeit kann vorgesehen sein, dass eine Positionierungsstruktur zur zumindest teilweisen Anordnung der Verpackung mit dem Lebensmittel in einem Anordnungsbereich als Messbereich vorgesehen ist, wobei eine Sendeeinheit und eine Empfangseinheit der Erfassungsanordnung durch die Positionierungsstruktur auf den Messbereich ausgerichtet sind. Bspw. können die Sensoren (es bilden z. B. die Empfangs- und Sendeeinheit einen Sensor) der Erfassungsanordnung an der Positionierungsstruktur befestigt sein, damit eine Positionierung und/oder Anordnung und/oder Ausrichtung der Sensoren an der Verpackung bzw. dem Anordnungsbereich möglich ist. Außerdem kann die Positionierungsstruktur eine Halteeinheit aufweisen, um eine Ausrichtung und/oder lösbare Befestigung der Verpackung an der Positionierungsstruktur zu ermöglichen. Von Vorteil ist es, wenn der Messbereich die Messstrecke umfasst, also die Strecke, die eine Messeinwirkung, insbesondere ein Laserstrahl, durch das Lebensmittel nimmt.

Auch kann durch die erwähnte Positionierungsstruktur erreicht werden, dass unterschiedliche Messungen in unterschiedlichen (vordefinierten) Positionen stattfinden können. Aus den jeweiligen Messergebnissen in den entsprechenden Positionen können dann weitere Messwerte (Erfassung von Zusatzinformationen) generiert werden oder Störeinflüsse kompensiert werden, um die Genauigkeit der zu untersuchenden Eigenschaft des Lebensmittels bzw. des Untersuchungsergebnisses zu verbessern.

Vorteilhaft ist es darüber hinaus, wenn bei dem Prüfgerät eine Kommunikationsvorrichtung vorgesehen ist, um eine, insbesondere drahtlose, Datenkommunikation mit einem mobilen Kommunikationsgerät und/oder einer zentralen Datenverarbeitungsanlage durchzuführen, um ein Ergebnis der Erfassung über die Datenkommunikation zu übertragen. Das Ergebnis der Erfassung (Erfassungsergebnis) kann bspw. ein Messwert des wenigstens einen Parameters oder dergleichen sein. Insbesondere ist dann eine Übertragung notwendig, um das Erfassungsergebnis separat vom Prüfgerät auszuwerten und ein Untersuchungsergebnis zu bestimmen. Alternativ oder zusätzlich kann die Auswertung durch z. B. eine Auswerteeinheit auch durch das Prüfgerät selbst erfolgen, sodass das Prüfgerät das Untersuchungsergebnis bereitstellt. Dadurch kann ggf. auf weitere Geräte verzichtet werden, um den technischen Aufwand zu reduzieren. Es ist jedoch für manche Anwendungen von Vorteil, wenn die Auswertung durch ein externes Gerät erfolgt, da dies aufwendigere Berechnungen und/oder eine zentrale Bereitstellung von Kalibrierungsinformationen (z. B. durch eine Datenbank) ermöglicht. Die (insbesondere drahtlose oder auch drahtgebundene) Kommunikation erfolgt dabei bspw. zwischen wenigstens zwei der nachfolgenden Vorrichtungen:
- der Kommunikationsvorrichtung des Prüfgeräts,
- einem mobilen Kommunikationsgerät, insbesondere Mobilfunkgerät, z. B. einem Mobiltelefon und/oder Smartphone,
- einer Datenverarbeitungsanlage, z. B. wenigstens einem Server einer sogenannten "Cloud".

Auch kann die Kommunikation vorteilhafterweise als eine visuelle Datenübertragung, z. B. mittels eines QR-Codes erfolgen. In diesem Fall kann die Kommunikationsvorrichtung, z. B. ein Display zur Anzeige eines QR-Codes aufweisen. Auch ist es denkbar, dass zum Empfang von Daten die Kommunikationsvorrichtung einen QR-Code-Scanner oder dergleichen aufweist.

Die Datenverarbeitungsanlage ist z. B. als wenigstens ein Computer ausgebildet, und umfasst z. B. auch wenigstens einen Datenspeicher, sodass durch die Datenverarbeitungsanlage wenigstens eine Datenbank bereitgestellt werden kann. Durch die Datenverarbeitungsanlage und/oder durch das mobile Kommunikationsgerät, also durch die externen Geräte, sind weitergehende und aufwendigere Auswertungen möglich, als dies bei dem Prüfgerät der Fall sein kann. Damit kann bei dem erfindungsgemäßen Prüfgerät auf eine aufwendige Auswerteelektronik verzichtet, und somit die Mobilität des Prüfgeräts verbessert und die Kosten reduziert werden.

Außerdem ist es von Vorteil, wenn eine Halteeinheit, vorzugsweise Positionierungseinheit, vorgesehen ist, um das Prüfgerät an der Verpackung lösbar zu befestigen. Die Halteeinheit, insbesondere Positionierungseinheit, kann bspw. eine konische Aufnahme oder dergleichen aufweisen, um eine formschlüssige Verbindung zur Verpackung, insbesondere zu einem Kopfbereich und/oder Verschluss der Verpackung, zu ermöglichen. Bspw. ist die Verpackung als Flasche ausgeführt, insbesondere als PET (Polyethylenterephthalat) Flasche oder als Glasflasche oder dergleichen ausgeführt, und weist entsprechend zumindest teilweise eine transparente Wandung (also einen transparenten Bereich) auf. Um nun eine Messung an dem Lebensmittel in der Verpackung durchführen zu können, ist eine Positionierung der Sensoren der Erfassungsanordnung derart notwendig, dass diese auf diesen transparenten Bereich ausgerichtet sind. Hierzu kann die Halteeinheit dienen, da diese eine vordefinierte und damit für die Erfassung bekannte Anordnung und Ausrichtung der Verpackung ermöglicht.

Bevorzugt kann im Rahmen der Erfindung vorgesehen sein, dass eine Halteeinheit vorgesehen ist. Diese kann z. B. als Verschlussgreifer (insbesondere ein sogenannter "Chuck") zur Drehmomentmessung ausgebildet sein. Auch kann das Prüfgerät und/oder die Halteeinheit wenigstens ein Mittel zur Erfassung eines Sauerstoffgehalts und/oder eines Gehalts anderer Gase und/oder eines Alkoholgehalts aufweisen. Insbesondere ist die Halteeinheit dazu ausgeführt, an einem Flaschenverschluss der als Flasche ausgebildeten Verpackung fixiert zu werden, wobei die Halteeinheit vorzugsweise einen Dreh- und/oder Schwenkmechanismus aufweist. Insbesondere dient dabei die Halteeinheit bzw. der Chuck als Befestigungsmittel, sodass hierdurch eine Fixierung des erfindungsgemäßen Prüfgeräts an der Verpackung zuverlässig und sicher möglich ist. Die Befestigung des Prüfgeräts kann dabei an der Verpackung (Container) mit der Halteeinheit als Zentriereinheit erfolgen, ggf. an einem sogenannten "PET Neckring" oder dergleichen. Vorzugsweise kann auch eine definierte fixierbare Befestigung am Gebinde im Bereich des Gasraums (Kopfraums) der Verpackung zur Fixierung von der Halteeinheit genutzt werden. Vorteilhaft ist es darüber hinaus, wenn die Halteeinheit zur Erfassung eines Drehmoments beim Öffnen der Verpackung dient, da dieses einen Rückschluss auf die zu untersuchende Eigenschaft, insbesondere einen CO₂-Gehalt, ermöglicht. Hierzu kann bspw. ein Deckel (als Verschluss) der Verpackung langsam z. B. manuell geöffnet werden. Aufgrund der Befestigung der Halteeinheit am Verschluss bzw. Deckel erfolgt somit eine Kraftausübung an der Halteeinheit, insbesondere als eine Torsionskraft, welche durch ein Drehmomentmesssystem bei der Halteeinheit erfasst und/oder gemessen werden kann. Die hierbei ermittelten Messwerte können dann ggf. auch zur Bestimmung eines Untersuchungsergebnisses und/oder zur Bestimmung der zu untersuchenden Eigenschaft automatisiert ausgewertet werden. Insbesondere kann die (das Produkt zerstörende) Drehmomenterfassung durch das Drehmomentmesssystem zusätzlich und sekundär zur (zerstörungsfreien) optischen Erfassung des Parameters mittels der Sende- und Empfangseinheit und/oder des Temperatursensors erfolgen, bspw. um bei einem kritischen Untersuchungsergebnis der optischen Erfassung dieses kritische Ergebnis zu bestätigen.

Es kann weiter möglich sein, dass eine Halteeinheit zur Adaption an einen Kopfraum einer als Flasche ausgebildeten Verpackung ausgebildet ist, vorzugsweise um das Prüfgerät konzentrisch an einem Flaschenverschluss zu befestigen. In anderen Worten kann der Flaschenverschluss im Kopfraum einen Verschluss bzw. Deckel für die Flasche bilden, welche regelmäßig vordefinierte und damit bekannte Geometrien aufweist. Somit kann die Halteeinheit von vornherein an diese Geometrie angepasst sein, und/oder ggf. auch adaptiv für verschiedene Geometrien anpassbar ausgestaltet sein, z. B. durch einen Verstellmechanismus.

Vorteilhaft ist es darüber hinaus, wenn im Rahmen der Erfindung eine Halteeinheit vorgesehen und als Zentriereinheit ausgebildet ist, sodass eine Zentrierung des Prüfgeräts an der Verpackung in einer Messposition durch die Halteeinheit erfolgt. Die Messposition dient dabei zur Erfassung des wenigstens einen Parameters, und bietet daher eine zur Erfassung vorausgesetzte Positionierung der Verpackung. Damit kann die Erfassung, insbesondere als Messung, zuverlässig ermöglicht werden.

Außerdem ist es von Vorteil, wenn die Sendeeinheit derart auf die Empfangseinheit und einen Anordnungsbereich ausgerichtet ist, dass eine Messeinwirkung zunächst in den Anordnungsbereich und dann zur Empfangseinheit gelangt, um eine Veränderung der Messeinwirkung durch das Lebensmittel zu erfassen. Die Messeinwirkung ist eine Laserstrahlung, welche durch eine Wandung der Verpackung zum Lebensmittel gelangt, und durch die Moleküle des Lebensmittels verändert werden (z. B. durch eine Absorption). Diese Veränderung kann wellenlängenabhängig sein, und es können ggf. Verbreiterungsmechanismen genutzt werden, sodass die Veränderung anhand eines Absorptionsspektrums ausgewertet werden kann. Das Absorptionsspektrum wird bspw. durch die Empfangseinheit dadurch erfasst, dass während der Erfassung die Wellenlänge der emittierten Laserstrahlung automatisiert verändert wird.

Weiter ist es bei dem erfindungsgemäßen Prüfgerät möglich, dass wenigstens eine Lichtquelle, insbesondere in Form eines durchstimmbaren Dioden-Lasers, bei der Erfassungsanordnung vorgesehen ist, und eine Messeinwirkung als ausgestrahltes Licht der Lichtquelle durch einen optischen Sensor messtechnisch erfassbar ist, um eine CO₂-Messung bei dem Lebensmittel durchzuführen. Dabei kann für einen Betrieb der durchstimmbaren Lichtquelle eine Wellenlänge des emittierten Lichts verändert werden. Insbesondere kann die Lichtquelle als ein Infrarotlaser und der Sensor als ein Infrarotsensor ausgeführt sein. Somit kann eine variierende Wellenlänge im Infrarotbereich emittiert und erfasst werden.

Zudem ist im Rahmen der Erfindung denkbar, dass eine Anzeigevorrichtung zur Darstellung eines maximal dreistufigen oder maximal vierstufigen Untersuchungsergebnisses vorgesehen ist. Die Anzeigevorrichtung dient insbesondere zur Anzeige eines Untersuchungsergebnisses, vorzugsweise einer Anzeige der Messergebnisse. Alternativ oder zusätzlich kann die Anzeige auf einem Anzeigemittel eines mobilen Kommunikationsgeräts erfolgen. Die Anzeigevorrichtung und/oder das Anzeigemittel ist bspw. als elektronisches Display und/oder als Touchscreen und/oder dergleichen ausgebildet.

Es kann weiter möglich sein, dass für einen portablen Betrieb des Prüfgeräts ein Energiespeicher, vorzugsweise ein Akkumulator, zur mobilen Energieversorgung vorgesehen ist. Hierbei kann der Energiespeicher derart kompakt ausgebildet sein, dass eine Anordnung in einem Gehäuse des Prüfgeräts möglich ist. Dadurch wird die Ausbildung als Handheld in einfacher Weise ermöglicht.

Auch ist es optional denkbar, dass die Erfassungsanordnung einen optischen Temperatursensor, insbesondere ein Bolometer oder Pyrometer, aufweist, um eine Temperatur bei dem Lebensmittel in der Verpackung zu erfassen. Darunter wird auch verstanden, dass eine Temperatur an einer Oberfläche der Verpackung erfasst wird. Es kann möglich sein, dass der Temperatursensor zur Erfassung der Temperatur auf einen Bereich der Verpackung unterhalb einer Gasphase des Lebensmittels und/oder unterhalb eines Füllstands des Lebensmittels in der Verpackung ausgerichtet ist, und entsprechend die Temperatur in diesem Bereich des Lebensmittels in der Flüssigkeit erfasst. Der Temperatursensor kann zur Erfassung eine Lichtquelle, vorzugsweise eine Laserquelle, und/oder einen optischen Sensor aufweisen.

Ebenfalls Gegenstand der Erfindung ist ein System zur Untersuchung, insbesondere zur zerstörungsfreien Druck- und Temperaturerfassung, vorzugsweise zur CO₂-Erfassung, bei Lebensmitteln in Verpackungen, aufweisend:
- ein, insbesondere erfindungsgemäßes, Prüfgerät zur Erfassung wenigstens eines Parameters bei einem Lebensmittel in einer Verpackung, wobei der Parameter für eine zu untersuchende Eigenschaft des Lebensmittels spezifisch ist,
- eine Auswerteeinheit zur Auswertung der Erfassung und insbesondere wenigstens einer weiteren Information, wie einer Kalibrierungsinformation, um die zu untersuchende Eigenschaft des Lebensmittels zu bestimmen, wobei das Prüfgerät als ein erfindungsgemäßes Prüfgerät ausgebildet ist.

Damit bringt das erfindungsgemäße System die gleichen Vorteile mit sich, wie sie ausführlich mit Bezug auf ein erfindungsgemäßes Prüfgerät beschrieben worden sind. Zudem kann das System ein erfindungsgemäßes Prüfgerät aufweisen.

Bevorzugt ist dabei das Prüfgerät dazu ausgebildet, von außen an der Verpackung angeordnet zu werden, um die Erfassung in zerstörungsfreier Weise durchzuführen. In anderen Worten kann die Verpackung verschlossen bleiben, und somit die Erfassung von Parametern des Lebensmittels indirekt durch die Verpackung erfolgen.

Bspw. kann bei dem erfindungsgemäßen System die Auswerteeinheit als ein elektronischer Prozessor ausgebildet sein, welcher optional mit einem Datenspeicher verbunden ist, um die Auswertung als eine Datenverarbeitung durchzuführen. Weiter kann anhand der Erfassung, also an zumindest einem Ergebnis der Erfassung bzw. Messwert, durch die Auswertung und ggf. anhand von wenigstens einer Kalibrierungsinformation eine Information über die zu untersuchende Eigenschaft als Untersuchungsergebnis bestimmt werden. Dies ermöglicht es z. B. eine Qualität des Lebensmittels einfach, kostengünstig und flexibel zu untersuchen.

Vorzugsweise kann vorgesehen sein, dass die Auswerteeinheit Teil des Prüfgeräts ist, sodass die Auswertung durch das Prüfgerät durchgeführt wird. Damit ist die Auswertung kompakt und kostengünstig ohne weitere Hilfsmittel möglich.

Es kann von Vorteil sein, wenn im Rahmen der Erfindung ein mobiles Kommunikationsgerät vorgesehen ist, welches die Auswerteeinheit umfasst, um die Auswertung separat vom Prüfgerät durchzuführen. Bspw. ist das Kommunikationsgerät als ein Smartphone ausgebildet, und weist deshalb einen leistungsfähigen Prozessor zur Auswertung auf. Es kann dabei auch möglich sein, dass die Auswertung zur Bestimmung eines Untersuchungsergebnisses nur teilweise durch die Auswerteeinheit durchgeführt wird, und ggf. noch eine weitere Auswerteeinheit (z. B. einer Datenverarbeitungsanlage, wie eines Servers) genutzt wird. Hierzu kann eine vernetzte Auswertung vorgesehen sein, welche eine Kommunikation z. B. mit einer Cloud nutzt.

Vorteilhaft ist es zudem, wenn ein mobiles Kommunikationsgerät vorgesehen ist, welches wenigstens ein Sensorelement aufweist, um eine Information über das Lebensmittel in der Verpackung und/oder über die Verpackung (insbesondere eine produktspezifische Information) separat vom Prüfgerät zu erfassen, sodass die Untersuchung, insbesondere die Bestimmung der zu untersuchenden Eigenschaft des Lebensmittels, in Abhängigkeit von der erfassten Information und des erfassten Parameters erfolgt. Bspw. ist das Sensorelement des Kommunikationsgeräts als ein Bildsensor ausgeführt, um eine Bildinformation (Foto) des Lebensmittels in der Verpackung aufzunehmen. Durch eine Analyse dieser Bildinformation, z. B. durch das Kommunikationsgerät selbst oder durch eine Datenverarbeitungsanlage, kann dann wenigstens eine weitere Information über das Lebensmittel in der Verpackung und/oder über die Verpackung (insbesondere eine produktspezifische Information) ermittelt werden. Eine solche Information ist z. B. eine Information über die Art des Lebensmittels und/oder eine Information über die Verpackung (Geometrie, Aufdruck oder dergleichen). Anhand der durch das Kommunikationsgerät erfassten Information kann in anderen Worten also eine Klassifizierung erfolgen, um das zu untersuchende Produkt zu bestimmen. Alternativ oder zusätzlich (z. B. bei fehlgeschlagener Klassifizierung) kann auch eine manuelle Auswahl der Information, z. B. aus einer Liste, ggf. bei dem Kommunikationsgerät erfolgen. Diese Information kann dann genutzt werden, um die Erfassung und/oder Auswertung durchzuführen, bspw. um wenigstens eine Kalibrierungsinformation auszuwählen.

Auch kann es möglich sein, dass das Prüfgerät ein Anzeigemittel zum Anzeigen eines Codes, vorzugsweise Barcodes und/oder QR-Codes, und/oder einen Bildsensor, wie einen Scanner zum Scannen eines solchen Codes aufweist. Das Anzeigemittel und/oder der Bildsensor können z. B. Teil einer Kommunikationsvorrichtung des Prüfgeräts sein, um einen Datenaustausch zu bewirken, z. B. um Daten mit einem Kommunikationsgerät oder dergleichen auszutauschen. Das Kommunikationsgerät ist bspw. ein Smartphone oder eine Kamera oder dergleichen, welche zur Auswahl und/oder Erfassung von wenigstens einer Information über das Lebensmittel in der Verpackung und/oder über die Verpackung dienen kann. Auch kann das Kommunikationsgerät dazu dienen, die Untersuchung anhand der erfassten Parameter zu initiieren und/oder eine Auswertung der Erfassung durchzuführen und/oder zu initiieren. Hierzu können die erfassten Parameter (z. B. die Messwerte) z. B. von dem Prüfgerät an das Kommunikationsgerät oder auch an ein anderes Gerät zur Auswertung mittels des Datenaustausches übertragen werden. In anderen Worten kann das Anzeigemittel dazu ausgeführt sein, ein Ergebnis der Erfassung der Parameter (insbesondere kodiert) anzuzeigen. Es kann sich bei diesem Ergebnis z. B. um wenigstens einen Messwert einer beim Lebensmittel erfassten Temperatur und/oder eines erfassten Drucks handeln.

Ebenfalls Gegenstand der Erfindung ist ein Verfahren zur Untersuchung, insbesondere zur zerstörungsfreien Druck- und Temperaturerfassung, vorzugsweise zur CO₂-Erfassung, bei Lebensmitteln in Verpackungen.

Hierbei ist insbesondere vorgesehen, dass die nachfolgenden Schritte durchgeführt werden, vorzugsweise nacheinander oder in beliebiger Reihenfolge, wobei einzelne Schritte ggf. auch wiederholt ausgeführt werden können:
a) Mobiles oder stationäres Erfassen von wenigstens einem Parameter bei einem Lebensmittel in einer Verpackung durch ein tragbares Prüfgerät, wobei der Parameter für eine zu untersuchende Eigenschaft des Lebensmittels spezifisch ist,
b) Auswerten zumindest der Erfassung des wenigstens einen Parameters, um ein Untersuchungsergebnis zu bestimmen,
c) Ausgeben des Untersuchungsergebnisses, wobei
ein erfindungsgemäßes Prüfgerät und/oder ein erfindungsgemäßes System betrieben wird.

Damit bringt das erfindungsgemäße Verfahren die gleichen Vorteile mit sich, wie sie ausführlich mit Bezug auf ein erfindungsgemäßes Prüfgerät und/oder ein erfindungsgemäßes System beschrieben worden sind. Zudem kann das erfindungsgemäße Verfahren zum Betreiben eines erfindungsgemäßen Systems und/oder eines erfindungsgemäßen Prüfgeräts dienen.

Die Bestimmung des Untersuchungsergebnisses und insbesondere eines CO₂-Gehalts des Lebensmittels kann vorteilhafterweise anhand wenigstens eines Messwertes der Erfassung erfolgen, z. B. durch das Prüfgerät oder durch ein externes Gerät.

Vorteilhafterweise kann zur Auswertung und/oder zur Bestimmung des CO₂-Gehalts ein (theoretisches, mathematisches, chemisches und/oder physikalisches) Modell oder es können verschiedene (theoretische, mathematische, chemische und/oder physikalische) Modelle und/oder eine von der Erfassung abhängige Parametrisierung des wenigstens einen Modells genutzt werden. Die Auswahl des Modells und/oder die Parametrisierung kann dabei von der Geometrie der Verpackung und/oder von einem Durchmesser eines Flaschenhalses der Verpackung sowie ggf. dem Lebensmittel selbst abhängig sein.

Vorzugsweise kann eine Geometrie der Verpackung, insbesondere einer Flasche, genutzt werden, um insbesondere eines von mehreren (z. B. mindestens 5 oder mindestens 10) Modellen für die Auswertung auszuwählen. Hierzu kann bspw. eine Eingabe über ein Eingabemittel wie ein Touchscreen des Prüfgeräts erfolgen. Auch kann für diese Auswahl ggf. ein Datenaustausch z. B. mittels QR-Codes erfolgen.

Die Erfassung oder mehrere Erfassungen des wenigstens einen Parameters bei einem erfindungsgemäßen Prüfgerät und/oder bei einem erfindungsgemäßen Verfahren, insbesondere die Erfassung und/oder wiederholte Erfassungen gemäß Schritt a), kann (können) insbesondere durch die Erfassungsanordnung an einer statischen Position an der Verpackung (vorzugsweise an einem Flaschenhals z. B. durch einen oder mehrere Sensoren) oder alternativ an mehreren Positionen an der Verpackung (z. B. durch den gleichen Sensor mittels einer Bewegung des Sensors, des Prüfgeräts oder der Verpackung) durchgeführt werden. Mit anderen Worten ist es auch möglich, dass mehrere Erfassungen an unterschiedlichen (ggf. vordefinierten) Positionen nacheinander oder gleichzeitig erfolgen. Bei der Nutzung von mehreren Positionen, an welchen die Erfassung erfolgt, kann z. B. das Prüfgerät (z. B. aufgrund der Positionierungsstruktur) und die Verpackung relativ zueinander während der Durchführung der Erfassungen bewegt werden. Auf diese Weise kann anhand der Erfassung eine Zusatzinformation ermittelt werden, welche beispielsweise für eine Geometrie der Verpackung spezifisch ist. Insbesondere kann anhand der Erfassung die Geometrie der Verpackung ermittelt werden, wenn bei der Auswertung die Positionen der Erfassungen berücksichtigt werden. Bspw. ist ein Ergebnis (Messwert) der Erfassung und/oder das ermittelte Spektrum von der Geometrie abhängig. Zur vereinfachten Relativbewegung zwischen Messeinrichtung und Verpackung ist diese z. B. als gabelförmiges Prüfgerät oder dergleichen ausgebildet. Das Prüfgerät kann eine Ausnehmung zur Aufnahme und/oder Verschiebung und/oder Bewegung der Verpackung (insbesondere des Flaschenhalses) für mehrere Positionen innerhalb der Ausnehmung aufweisen. Die ermittelte Geometrie kann z. B. bei der Auswertung berücksichtigt werden. Insbesondere erfolgt bei dem erfindungsgemäßen Verfahren eine Erfassung eines Gesamtdrucks in einem Kopfraum der Verpackung und/oder eine Erfassung eines Partialdrucks eines Gases (z.B. CO₂) der Gasphase in der Verpackung. Bspw. kann zur Auswertung und Bestimmung der Eigenschaft des Lebensmittels gemäß dem physikalischen Gesetz von Henry & Dalton der in der Flüssigkeit gelöste CO₂-Gehalt bestimmt werden. Hierzu kann bspw. zumindest eine arithmetische Berechnung durch die Auswerteeinheit und/oder durch das mobile Kommunikationsgerät und/oder durch die Datenverarbeitungsanlage durchgeführt werden. Bevorzugt wird der Druck anhand eines Spektrums, vorzugsweise Absorptionsspektrums, einer Messeinwirkung, insbesondere elektromagnetischer Wellen (z.B. Licht- bzw. Laserstrahls), erfasst. Dies kann eine Auswertung einer Linienverbreitung umfassen, wobei diese u.a. von einem Absolutdruck des Prüfobjekts abhängig ist. Je höher der Gasdruck, desto flacher und breiter werden die Absorptionskurven. Die Formbestimmung der Absorptionskurve kann hierbei einen Rückschluss auf den Gesamtdruck erlauben. In anderen Worten kann bei der Erfassung und/oder Auswertung gemäß dem erfindungsgemäßen Verfahren der Gesamtdruck anhand einer Kurvenanalyse im Spektrum erfasst werden. Welche Parameter für die Auswertung genutzt werden ist dabei bspw. von wenigstens einer Kalibrierungsinformation (also den Kalibrierungsdaten) abhängig.

Des Weiteren kann es möglich sein, dass eine optische (insbesondere zerstörungsfreie) Bestimmung von einem Druck und/oder einer Temperatur bei dem Lebensmittel erfolgt. Alternativ oder zusätzlich kann durch die Untersuchung gemäß dem erfindungsgemäßen Verfahren eine Transparenzprüfung für die Verpackung und/oder eine Geometrieerfassung und/oder eine Lagebestimmung an der Verpackung und/oder eine Drehmomentmessung bereitgestellt werden. Es kann z. B. auch eine dynamische Transparenzkontrolle der Messstrecke (z. B. zur Erkennung von Schaum und/oder Aufklebern) durch das erfindungsgemäße Prüfgerät möglich sein.

Ferner kann es im Rahmen des erfindungsgemäßen Verfahrens vorgesehen sein, dass das Untersuchungsergebnis maximal dreistufig gebildet wird, vorzugsweise gemäß einer Ampelabstufung, wobei bevorzugt hierzu ein Zwischenergebnis der Auswertung mit zumindest zwei verschiedenen Schwellenwerten verglichen wird, sodass eine dreistufige Einordnung anhand des Vergleichs erfolgt. Bspw. kann dabei diese Einstufung anhand der wenigstens einen Kalibrierungsinformation erfolgen, sodass die Einstufung in Abhängigkeit von dem Lebensmittel in der Verpackung durchgeführt wird. Dies hat den Hintergrund, dass für unterschiedliche Lebensmittel unterschiedliche Grenzwerte zur Bestimmung der Qualität genutzt werden können. Insbesondere umfasst daher die wenigstens eine Kalibrierungsinformation den wenigstens einen Schwellenwert zur Einordnung. Dabei ist es auch denkbar, dass Warngrenzen für die Produktqualität ausgewertet und/oder ausgegeben werden. Die Ausgabe gemäß Schritt c) erfolgt z. B. an einem externen Gerät, wie einen PC oder Tablet oder Telefon, oder auch direkt an dem Prüfgerät.

Optional kann es bei dem erfindungsgemäßen Verfahren vorgesehen sein, dass vor Schritt a) eine Information, vorzugsweise Bildinformation, durch ein optisches Sensorelement, vorzugsweise eines mobilen Kommunikationsgeräts, insbesondere eines Mobilfunkkommunikationsgeräts, über die Verpackung und/oder das Lebensmittel in der Verpackung erfasst wird, und bei Schritt b) das Auswerten auch anhand dieser Information erfolgt, bevorzugt um die Art der Verpackung und/oder des Lebensmittels zu berücksichtigen. Von Vorteil ist es, wenn das Sensorelement (z. B. als eine Optikeinheit) zur Erkennung der Verpackung und/oder des Produkts (der Probe) dient. Somit kann eine Information über das Produkt, wie z. B. über einen Barcode oder ein anderes Identifikationsmerkmal, wie ein Foto oder eine manuelle Produktauswahl, ermittelt werden, um die Art des Lebensmittels zu bestimmen, und bei der Auswertung zu berücksichtigen.

Ein weiterer Vorteil im Rahmen der Erfindung ist erzielbar, wenn vor Schritt a) wenigstens eine Information über die Verpackung und/oder über das Lebensmittel durch eine Kommunikation des Prüfgeräts oder eines mobilen Kommunikationsgeräts an eine externe Datenverarbeitungsanlage übermittelt wird, und anhand einer Datenbank der Datenverarbeitungsanlage wenigstens eine Kalibrierungsinformation in Abhängigkeit von der übermittelten Information ermittelt wird, um anhand der Kalibrierungsinformation die Erfassung gemäß Schritt a) und/oder die Auswertung gemäß Schritt b) durchzuführen. Es kann z. B. das Durchstimmen und/oder ein Kalibrieren bei der spektroskopischen Messung anhand der Kalibrierungsinformation erfolgen. Bevorzugt sind hierzu in der Datenbank Kalibrierungsdaten als produktspezifische Informationen und/oder Charakterisierungsparameter gespeichert, um die wenigstens eine Kalibrierungsinformation bereitzustellen. Die Kalibrierungsdaten umfassen z. B. Kalibrierkurven, welche für unterschiedliche Proben und/oder Produkte (Lebensmitteln in Verpackungen) angelegt werden. Das Prüfgerät erfasst daher z. B. nur Rohdaten, die Auswertung anhand der Kalibrierungsinformation kann dann separat vom Prüfgerät, also z. B. durch ein externes Gerät, erfolgen.

Außerdem kann es im Rahmen der Erfindung von Vorteil sein, dass Schritt a) und/oder b) und/oder c) autonom durch das Prüfgerät durchgeführt wird. Unter einer autonomen Durchführung ist dabei zu verstehen, dass externe Geräte nicht für die entsprechenden Schritte herangezogen werden müssen. Dies hat den Vorteil, dass eine schnelle und vereinfachte Durchführung möglich ist.

Ferner kann es im Rahmen der Erfindung vorgesehen sein, dass die Schritte b) und/oder c) zumindest teilweise durch ein externes Gerät, vorzugsweise durch die Datenverarbeitungsanlage und/oder durch das mobile Kommunikationsgerät, durchgeführt werden, wobei vorzugsweise hierzu eine Datenkommunikation des Prüfgeräts über ein Datennetzwerk mit dem externen Gerät erfolgt. Dies ermöglicht auch eine aufwendige Verarbeitung, bei welcher z. B. ein stationärer Rechner notwendig ist. Zumindest die Erfassung kann dabei jedoch mobil durch das tragbare Prüfgerät, z. B. als Handheld, erfolgen. Dies verbessert die Flexibilität des Verfahrens deutlich.

Nach einer weiteren Möglichkeit kann bei dem erfindungsgemäßen Verfahren vorgesehen sein, dass das Erfassen gemäß Schritt a) und/oder das Auswerten gemäß Schritt b) eine spektroskopische Untersuchung eines Lichtstrahls umfasst. Insbesondere kann dabei im Spektrum besonders zuverlässig der Parameter ermittelt werden.

Es ist ferner denkbar, dass vor Schritt b) wenigstens eine geometrische Eigenschaft der Verpackung messtechnisch erfasst wird, vorzugsweise durch ein mobiles Kommunikationsgerät, bevorzugt durch eine Bildaufnahme, und bei Schritt b) ausgewertet wird. Damit können weitergehende Information genutzt werden, wie z. B. die geometrische Eigenschaft, um z. B. in Abhängigkeit von dieser Information und/oder einer Klassifizierung anhand dieser Information wenigstens eine Kalibrierungsinformation auszuwählen.

Darüber hinaus ist es von Vorteil, wenn eine Information über die Verpackung bestimmt wird, und vorzugsweise bei Schritt b) eines erfindungsgemäßen Verfahrens diese Information mit einem Ergebnis der Erfassung kombiniert und/oder verglichen und/oder gemeinsam ausgewertet wird, um einen Druck in einem Kopfraum der Verpackung zu bestimmen. Bevorzugt erfolgt eine Berechnung eines CO₂-Gehalts anhand eines Ergebnisses der Auswertung gemäß Schritt b), wobei das Ergebnis dieser Berechnung als Untersuchungsergebnis gemäß Schritt c) dargestellt werden kann. Insbesondere wird die Information über die Verpackung (z. B. als Bildinformation) durch ein mobiles Kommunikationsgerät aufgenommen und dient zur Bestimmung von Eigenschaften der Verpackung, z. B. Typ, Material, Geometrie oder Flaschenhalsabmessungen. Diese Information und/oder die bestimmten Eigenschaften können dann mit dem Ergebnis der Erfassung gemäß Schritt a), also insbesondere den Laserdaten, zur Berechnung des Drucks im Kopfraum der Verpackung, insbesondere eines Partialdrucks und/oder eines Gesamtdrucks, genutzt werden.

Außerdem kann es im Rahmen der Erfindung von Vorteil sein, dass gemäß Schritt a) zumindest die Temperatur und/oder der Druck gemessen wird, um eine Gaskonzentration in der Verpackung aus den Messwerten dieser Messung zu bestimmen, vorzugsweise in Abhängigkeit von wenigstens einer Kalibrierungsinformation, welche in Abhängigkeit von dem Lebensmittel in der Verpackung (und/oder der Verpackung) ermittelt werden. Bspw. wird hierbei die wenigstens eine Kalibrierungsinformation in Abhängigkeit von einer Klassifizierung automatisiert ausgewählt, und ggf. alternativ oder zusätzlich manuell ausgewählt. Die Klassifizierung erfolgt dabei z. B. zur Bestimmung einer Art des Lebensmittels und/oder der Verpackung, und kann ggf. anhand einer produktspezifischen Information durchgeführt werden, welche durch wenigstens ein Sensorelement erfasst wird. Das Sensorelement ist bspw. in dem Prüfgerät oder im Kommunikationsgerät integriert und führt vorzugsweise eine messtechnische Erfassung und/oder eine Bildaufnahme des Lebensmittels in der Verpackung durch, vorzugsweise zerstörungsfrei (d. h. ohne Beschädigung und/oder ohne ein Öffnen der Verpackung).

Es ist weiter im Rahmen der Erfindung möglich, dass zur Bestimmung wenigstens einer Kalibrierungsinformation (Kalibrierungsdaten) wiederholt Referenzmessungen bei Proben durchgeführt werden. Insbesondere wird dabei wenigstens ein Messergebnis der jeweiligen Probenmessung dauerhaft gespeichert, und ggf. durch eine Datenbank bereitgestellt. Es empfiehlt sich für jedes Lebensmittelprodukt (gemeint ist das gleiche Lebensmittel, z.B. alkoholfreies Bier einer bestimmten Marke) eine eigene Referenzmessung, um somit exakte Referenzmessungen für eine spätere Vergleichsmessung zu erhalten. Unter der Vergleichsmessung wird dabei z. B. die Erfassung des wenigstens einen Parameters verstanden, wobei ein Ergebnis der Erfassung mit der Referenzmessung bei der Auswertung verglichen wird, um die Eigenschaft zu bestimmen.

Das vorliegende Verfahren kann für unterschiedliche Lebensmittel in unterschiedlichen Verpackungen durchgeführt werden. Dabei können für die unterschiedlichen Lebensmittel und/oder Verpackungen unterschiedliche Kalibrierungsinformationen bestimmt und in der Datenbank gespeichert werden. Auch kann es möglich sein, dass für die unterschiedlichen Lebensmittel bzw. Verpackungen jeweils ein Identifikator bestimmt und bereitgestellt wird. Bei einer Untersuchung kann durch ein Gerät, wie ein mobiles Kommunikationsgerät, eine Information über das Lebensmittel und/oder die Verpackung erfasst werden, bevor die Erfassung durch das erfindungsgemäße Prüfgerät erfolgt. Der Identifikator wird dann mit der Information über das Lebensmittel bzw. Verpackung, wie einer Bildinformation, verglichen, um das zu untersuchende Lebensmittel und/oder die Verpackung zu bestimmen. In Abhängigkeit von dieser Bestimmung kann sodann die Erfassung und/oder Auswertung durchgeführt werden, bei welcher zumindest eine in Abhängigkeit von dem Vergleich ausgewählte Kalibrierungsinformation genutzt wird.

Erfindungsgemäß kann es vorgesehen sein, dass zur Bestimmung wenigstens einer Kalibrierungsinformation eine direkte Probenmessung des Lebensmittels durch Zerstörung der Verpackung stattfindet. Hierbei ist es denkbar, dass die Verpackung z. B. durch ein Anstechmittel angestochen wird, um wenigstens einen Messsensor in die Verpackung einführen zu können. Durch diesen Messsensor ist es dann möglich, zumindest eine physikalische, chemische und/oder biologische Eigenschaft des Lebensmittels zu messen. Hierbei kann es sich z. B. um die Temperatur, den Druck, die Feuchtigkeit, den elektrischen Widerstand oder weitere Eigenschaften des Lebensmittels handeln, die direkt messbar sind. Die direkte Probenmessung weist damit den Vorteil auf, dass die Messergebnisse von dem Messsensor unter Ausschluss der Messumgebung und somit ohne Messfehler stattfindet. Damit kann eine exakte Probenmessung stattfinden, die eine hohe Genauigkeit der gemessenen Eigenschaft des Lebensmittels aufweist. Allerdings weist die direkte Probenmessung auch den Nachteil auf, dass die Verpackung i. d. R. nicht wieder verschlossen werden kann, sodass am Ende der Probenmessung das Lebensmittel mit der Verpackung unbrauchbar, insbesondere für spätere Messungen ist. Dies liegt einerseits daran, dass nach der Messung Luft in die Verpackung eindringt und andererseits, dass z. B. ein Überdruck oder ein Inertgas nach der direkten Probemessung entweichen kann. Außerdem wird die Kühlkette der gemessenen Lebensmittelprobe durch die Messung i. d. R. unterbrochen. Die hierdurch bestimmte wenigstens eine Kalibrierungsinformation kann dann ggf. für eine Vielzahl von weiteren Untersuchungen durch das Prüfgerät dienen, und damit zerstörungsfreie Prüfungen ermöglichen.

Ferner ist es im Rahmen der Erfindung denkbar, dass bei der Untersuchung durch das erfindungsgemäße Prüfgerät eine indirekte Probenmessung des Lebensmittels stattfindet, bei der eine zerstörungsfreie Untersuchung vorgenommen wird. Hierbei bleibt die Verpackung i. d. R. unversehrt, da eben die Probenmessungen ausschließlich durch die verschlossene Verpackung stattfinden. Dabei können insbesondere physikalische, chemische und/oder biologische Eigenschaften des Lebensmittels vorzugsweise kontaktlos (zum Lebensmittel) durch die Verpackung gemessen werden. Dieses kann z. B. durch optische, induktive, kapazitive und/oder elektromagnetische Erfassungen, insbesondere Messungen, vorzugsweise durch die Erfassungsanordnung erfolgen. Dabei können auch Röntgen- oder Ultraschallmessungen sowie magnetische Resonanzmessungen oder dergleichen zum Einsatz kommen.

Bei dem erfindungsgemäßen Verfahren kann es ferner vorgesehen sein, dass bei der Untersuchung zumindest die Temperatur- oder der Druck in der Verpackung gemessen wird. Durch die zuvor genannten und gemessenen Eigenschaften und ggf. die weiteren physikalischen, chemischen und/oder biologischen Eigenschaften des Lebensmittels lässt sich dann eine Folgerung über die Qualität und den Geschmack des Lebensmittels und/oder über das Mindesthaltbarkeitsdatum als Untersuchungsergebnis erzielen.

Ferner ist es im Rahmen der Erfindung denkbar, dass ein Gasgehalt in der Verpackung anhand der Erfassung, insbesondere aus Messwerten der Temperatur und des Druckes, ermittelt wird. Hierzu kann insbesondere eine Berechnungsfunktion dienen, die als Eingangswerte zumindest die Temperatur und den Druck in einer Verpackung benötigen und dann anhand dieser Eingangswerte den Gasgehalt in dem entsprechenden Lebensmittel bestimmen. Hierzu kann eine Auswerteeinheit genutzt werden. Bevorzugt wird für jedes Lebensmittel (z. B. Bier, Limonade usw.) eine entsprechende Berechnungsfunktion in Abhängigkeit von Temperatur und Druck ermittelt, mit deren Hilfe sich dann der entsprechende Gasgehalt in der Verpackung ermitteln lässt. Gerade bei Getränken, die im Flüssigkeitsbehälter oder Flaschen angeordnet sind, spielt der Gasgehalt (z.B. CO₂) eine wesentliche Rolle. Von Vorteil ist es ferner, wenn wenigstens eine Kalibrierungsinformation die entsprechende Berechnungsfunktion aufweist oder indiziert.

Im Rahmen der Erfindung ist es optional vorgesehen, dass z.B. der Gasgehalt zumindest in der Verpackung oder im Lebensmittel die Haltbarkeit wesentlich beeinflusst. Hierdurch wird durch den Gasgehalt (z. B. CO₂ bei Bier) auch das Mindesthaltbarkeitsdatum im Wesentlichen beeinflusst. Sollte sich somit der Gasgehalt im Laufe der Zeit in der Verpackung reduzieren, da z. B. ein Teil durch die Verpackung diffundiert, so nimmt auch die Haltbarkeit des Lebensmittels entsprechend über die Zeit ab. Damit ist es vorzugsweise notwendig, den Gasgehalt in der Verpackung und/oder in dem Lebensmittel regelmäßig in zeitlichen Abständen, beispielsweise in vordefinierten Zeitabständen Δt zu kontrollieren.

Bei den zuvor erwähnten Lebensmitteln kann es sich insbesondere um ein Getränk handeln, damit sind flüssige Lebensmittel gemeint. Hierbei ist es denkbar, dass das Getränk insbesondere CO₂-haltig ist, wodurch - wie bereits beschrieben - die Qualität und der Geschmack des Getränks im Wesentlichen beeinflusst wird. Somit ergibt sich aus dem CO₂ Gehalt auch ein eindeutiger Rückschluss auf die Qualität und den Geschmack des Getränkes und das zu erreichende Mindesthaltbarkeitsdatum.

Bei dem erfindungsgemäßen Verfahren kann es optional vorgesehen sein, dass in einem Schritt vor einer Erfassung des wenigstens einen Parameters durch das Prüfgerät die Verpackung mit dem Lebensmittel zumindest geschüttelt oder auf eine vordefinierte Temperatur gebracht wird, um einen Gleichgewichtszustand innerhalb der Verpackung zu erreichen. Der Gleichgewichtszustand innerhalb der Verpackung ist für eine exakte Probenmessung sowohl bei einer indirekten als auch bei einer direkten Messung von Bedeutung. Ansonsten kann es unter Umständen zu Messfehlern kommen. Insbesondere bei CO₂-haltigen Getränken stellt sich ein einheitlicher Partialdruck in dem Gasraum und dem Flüssigkeitsraum der Verpackung durch das Schütteln ein. Auch kann die Verpackung mit dem Lebensmittel vor der Erfassung durch das Prüfgerät auf eine vordefinierte Temperatur gebracht werden, um somit temperaturbedingte Messfehler direkt auszuschließen. Dabei empfiehlt es sich, dass auch innerhalb der Verpackung eine homogene Temperatur vorhanden ist.

Des Weiteren kann es erfindungsgemäß vorgesehen sein, dass in einem Schritt vor einer Erfassung durch das erfindungsgemäße Prüfgerät wenigstens eine geometrische Eigenschaft der Verpackung, bspw. messtechnisch, erfasst wird, bspw. durch das Prüfgerät oder ein hierzu externes Gerät, wie ein mobiles Kommunikationsgerät. Bei der genannten geometrischen Eigenschaft, kann es sich z. B. um die äußeren Abmaße der Verpackung, insbesondere im Messbereich, beispielsweise dem Flaschenhals oder dem Kopfraum handeln. So kann z. B. der Außendurchmesser eines Getränkeflaschenhalses messtechnisch erfasst werden. Auch die Wandstärke der Verpackung im Messbereich kann technisch gemessen werden, um somit eine exakte Messung innerhalb der Verpackung vornehmen zu können. Hier kann z. B. der Innendurchmesser eines Flaschenhalses im Messbereich bestimmt werden, durch den z. B. die Lauflänge des Lichtstrahles bestimmbar ist. Aber auch der Brechungsindex des Verpackungsmaterials der Verpackung kann z. B. messtechnisch erfasst werden. Zur Erfassung dieser geometrischen Eigenschaft kann z. B eine Bildinformation aufgenommen und ausgewertet werden. Insgesamt dient die Erfassung der geometrischen Eigenschaften der Verpackung dazu, eine indirekte Probenmessung zu ermöglichen, bzw. Messfehler aufgrund von geometrischen Verpackungstoleranzen auszuschließen.

Bei der Erfindung ist es ferner möglich, dass die Erfassung des wenigstens einen Parameters des Lebensmittels durch zumindest eine optische Messung durch die Verpackung stattfindet. Diese optische Messung muss nicht im sichtbaren Lichtbereich für Menschen stattfinden. So kann z. B. auch eine Messung im infraroten oder ultravioletten Strahlungsbereich stattfinden. Auch andere elektromagnetische Spektren des Lichtes sind dabei denkbar. Wie zuvor erwähnt worden ist, kann auch eine Messung durch Ultraschall oder Röntgenstrahlen stattfinden.

Des Weiteren ist es im Rahmen des erfindungsgemäßen Verfahrens denkbar, dass die Verpackung zumindest teilweise (licht-)durchsichtig ausgestaltet ist oder zumindest ein optisch durchsichtiges Messfenster aufweist. Dabei muss dieser optisch durchsichtige Bereich nicht für das menschliche Auge durchsichtig sein, sondern nur für die zuvor beschriebenen optischen Messungen durchdringbar sein, um somit möglichst ohne Messfehler auf optische Art und Weise die Eigenschaften des Lebensmittels in der Verpackung messtechnisch erfassen zu können. Es versteht sich von selbst, dass ein entsprechender optischer Sensor geometrisch im optisch durchsichtigen Bereich der Verpackung misst. Hierzu kann auf einer gegenüberliegenden Seite des optischen Sensors eine entsprechende Lichtquelle, welche die optische Strahlung aussendet, angeordnet sein. Selbstverständlich sind auch mehrere optische Sensoren sowie Lichtquellen zur Durchführung des erfindungsgemäßen Verfahrens denkbar, die auch nicht alle in dem gleichen Frequenzspektrum, bzw. der gleichen Wellenlänge funktionieren müssen. Im Gegenteil, durch Lichtquellen, die zumindest zeitweise unterschiedliches Licht von unterschiedlichen Wellenlängen aussenden, kann eine optische Messung erleichtert werden. Auch monochromatisches, polarisiertes und/oder gepulstes Licht usw. kann zur optischen Messung verwendet werden.

Bei dem erfindungsgemäßen Verfahren kann es zusätzlich denkbar sein, dass die Verpackung das Lebensmittel druckdicht aufnimmt und umschließt. Hierbei kann die Verpackung insbesondere als Flasche oder Flüssigkeitsbehälter ausgestaltet sein. Üblicherweise weist eine derartige Verpackung einen Deckel auf, durch den die Flüssigkeit, insbesondere in Form eines Getränkes, aus der Verpackung ausgeschüttet werden kann.

Im Rahmen des erfindungsgemäßen Prüfgeräts ist es ferner denkbar, dass eine Halteeinheit eine mechanische Aufnahme für die Verpackung aufweist, und ggf. gleichzeitig mit einem Dreh- und/oder Schwenkmechanismus ausgestaltet ist. Durch diesen Dreh- und/oder Schwenkmechanismus kann die Verpackung geschüttelt werden, um einen Gleichgewichtszustand innerhalb der Verpackung herzustellen und damit eine exakte Probenmessung zu erzielen.

Ferner kann es bei dem erfindungsgemäßen Prüfgerät vorgesehen sein, dass eine Halteinheit, insbesondere als eine mechanische Aufnahme, für die Verpackung gleichzeitig mit dem Dreh-und/oder Schwenkmechanismus ausgestaltet ist. Somit kann die Verpackung durch die mechanische Aufnahme sicher an dem Dreh- und/oder Schwenkmechanismus fixiert werden. Folglich wird die mechanische Aufnahme durch die von dem Dreh- oder Schwenkmechanismus mitgedreht, wodurch dann ebenfalls die Verpackung mit dem Lebensmittel bewegt wird. Zu diesem Zweck kann an dem Prüfgerät ein entsprechender Antriebsmotor vorgesehen sein, der den Dreh- und/oder Schwenkmechanismus elektromechanisch antreibt.

Bei dem erfindungsgemäßen Prüfgerät ist es ferner denkbar, dass zumindest einer der nachfolgenden Sensoren vorhanden ist: Temperatursensor, Drucksensor, optischer Sensor, Gewichtssensor, Feuchtigkeitssensor, kapazitiver- oder induktiver Sensor, Widerstandssensor und dergleichen, jeweils insbesondere als Teil der Erfassungsanordnung. Auch sogenannte Kombisensoren, die mehrere Eigenschaften messen können, können zur Anwendung kommen.

Zum Beispiel ist eine Kommunikationsvorrichtung des Prüfgeräts als eine Bluetooth-Schnittstelle oder als eine serielle Schnittstelle oder als eine USB-Schnittstelle oder als eine Barcode- und/oder QR-Code Schnittstelle (bspw. als Scanner) oder als eine akustische Schnittstelle oder als eine NFC-Schnittstelle oder als eine Netzwerkschnittstelle (z. B. WLAN) oder als eine Mobilfunkschnittstelle oder dergleichen ausgebildet, um eine entsprechende (insbesondere drahtlose) Kommunikation des Prüfgeräts mit einem mobilen Kommunikationsgerät und/oder einer Datenverarbeitungsanlage und/oder dergleichen zu ermöglichen, bspw. auch über eine Internetverbindung. Über diese Kommunikation kann bspw. ein Erfassungsergebnis vom Prüfgerät übertragen werden und/oder ein Untersuchungsergebnis an das Prüfgerät übertragen werden. Die Kommunikation ist z. B. uni- oder bidirektional ausgeführt. Alternativ oder zusätzlich ist eine Eingabevorrichtung bei dem Prüfgerät vorgesehen, um das Prüfgerät zu bedienen (z. B. eine Information über das Lebensmittel und/oder die Verpackung zur Bestimmung der Kalibrierungsinformation manuell auszuwählen). Hierzu umfasst die Eingabevorrichtung z. B. mindestens einen Taster und/oder einen Touchscreen und/oder eine Sprachschnittstelle und/oder dergleichen.

Des Weiteren kann es bei dem erfindungsgemäßen Prüfgerät und/oder bei einem (dazu) externen Gerät, wie einem mobilen Kommunikationsgerät, möglich sein, dass ein Sensorelement zur Erfassung einer Information über das Lebensmittel und/oder die Verpackung vorgesehen ist. Das Sensorelement ist bspw. als Bildsensor oder als NFC- oder RFID-Sensor oder dergleichen ausgeführt, um bspw. eine Art des Lebensmittels oder der Verpackung zu erfassen. Hierzu können Eigenarten der Verpackung (wie ein Aufdruck oder ein Transponder) ausgewertet werden.

Im Rahmen des erfindungsgemäßen Prüfgeräts ist es vorgesehen, dass wenigstens eine Lichtquelle (Strahlungsquelle) in Form eines Lasers vorhanden ist und dass das ausgestrahlte Licht von einer Lichtquelle von einem optischen Sensor messtechnisch erfassbar ist. Selbstverständlich können auch mehrere Lichtquellen in dem Prüfgerät geometrisch zueinander angeordnet sein, um somit auch eine Messstrecke, bzw. ein Messfeld optisch messtechnisch zu erfassen. Als optische Sensoren können hier sogenannte Zeilensensoren oder Arraysensoren ausgestrahltes Licht der Lichtquelle messtechnisch erfassen. Eine Steuerung der Lichtquelle sowie eine Auswertung der erfassten messtechnischen Signale von dem oder den optischen Sensoren kann durch die Auswerteeinheit in dem Prüfgerät stattfinden. Gleichzeitig können die erhaltenen optischen Messdaten mit Messdaten eines Sensors, der innerhalb der Verpackung angeordnet ist, verglichen, verarbeitet und/oder gespeichert werden. Selbstverständlich kann jedoch auch die optische Messung getrennt von der direkten Probemessung stattfinden.

Ferner ist es erfindungsgemäß denkbar, dass das erfindungsgemäße Prüfgerät wenigstens eine Temperiereinheit zum Temperieren der Verpackung mit dem Lebensmittel aufweist. Dabei dient die Temperiereinheit dazu, die Verpackung mit dem Lebensmittel auf eine vordefinierte Temperatur zu bringen, sodass Messfehler aufgrund von unterschiedlichen Temperaturen verhindert werden. Außerdem ist es somit möglich, dass zumindest bei einer indirekten, zerstörungsfreien Probenmessung die Kühlung des Lebensmittels während der Messung nicht unterbrochen wird.

Weitere Vorteile, Merkmale und Einzelheiten der Erfindung ergeben sich aus der nachfolgenden Beschreibung, in der unter Bezugnahme auf die Zeichnungen Ausführungsbeispiele der Erfindung im Einzelnen beschrieben sind. Dabei können die in den Ansprüchen und in der Beschreibung erwähnten Merkmale jeweils einzeln für sich oder in beliebiger Kombination erfindungswesentlich sein. Es zeigt:

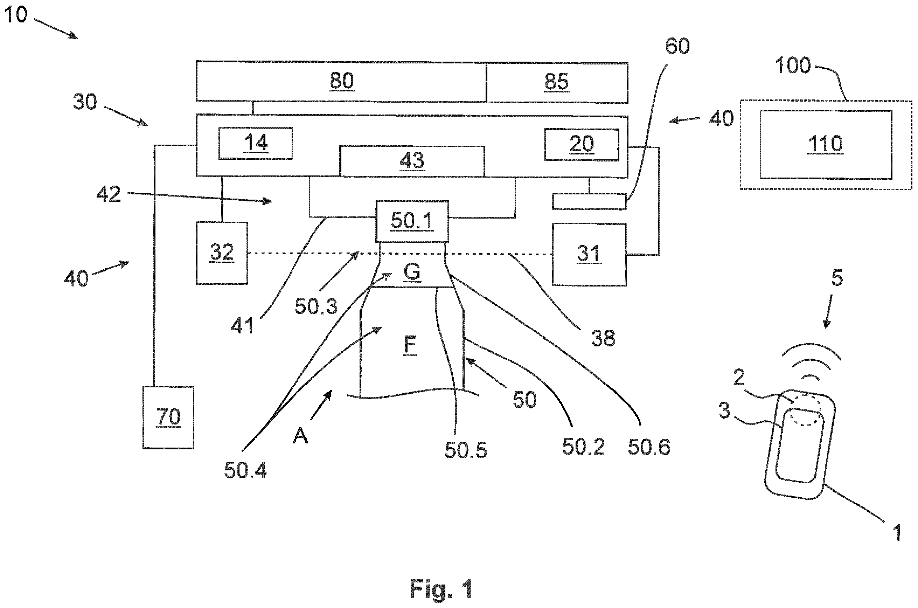

- Fig. 1: eine schematische Darstellung eines erfindungsgemäßen Systems mit einem erfindungsgemäßen Prüfgerät.

In Figur 1 ist schematisch ein erfindungsgemäßes System mit einem erfindungsgemäßen Prüfgerät 10 dargestellt. Dabei dient das System zur Untersuchung von Lebensmitteln 50.4 in Verpackungen 50, vorzugsweise in Flaschen 50.2. Hierzu kann das System das Prüfgerät 10 und/oder ein mobiles Kommunikationsgerät 1, wie ein Smartphone oder dergleichen, und/oder eine Datenverarbeitungsanlage 100, insbesondere einen externen Server 100, aufweisen.

Im gezeigten Ausführungsbeispiel ist das Lebensmittel 50.4 beispielhaft als Getränk ausgeführt, welches in einer Flasche 50.2 als Verpackung 50 enthalten ist. Hierbei ist auch ein Füllstand 50.5 dargestellt, wobei oberhalb des Füllstands 50.5 sich in der geschlossenen Flasche 50.2 eine Gasphase G und unterhalb des Füllstands 50.5 eine Flüssigkeit F befindet. Eine Wandung der Flasche 50.2 ist z. B. zumindest teilweise transparent ausgebildet. Im Kopfraum 50.3 kann ein Verschluss 50.1, insbesondere ein Deckel 50.1, angeordnet sein, um die Flasche 50.2 luftdicht zu verschließen. Des Weiteren ist ein Flaschenhals 50.6 dargestellt, in welchem sich zumindest überwiegend die Gasphase G befindet.

Bei Lebensmitteln 50.4 in Verpackungen 50 ist ein Problem, dass sich eine Untersuchung von wenigstens einer Eigenschaft des Lebensmittels 50.4, z. B. eines CO₂/ N2-Gehalts, nur aufwendig ermitteln lässt. Häufig muss dabei ein Öffnen der Verpackung 50 erfolgen, z. B. bei einer Ermittlung eines Drehmoments bei dem Verschluss 50.1 (bzw. dem Deckel 50.1) der Verpackung 50.

Daher kann erfindungsgemäß ein Prüfgerät 10 genutzt werden, welches eine zerstörungsfreie (insbesondere optische) Untersuchung ermöglicht. Hierzu ist eine Positionierungsstruktur 40 vorgesehen, mit welcher u. a. eine Erfassungsanordnung 30, insbesondere Messanordnung 30 zumindest zur Druckmessung, mechanisch verbunden ist. Dies hat den Vorteil, dass durch die Positionierungsstruktur 40 eine Anordnung und/oder Ausrichtung der Erfassungsanordnung 30 an der Verpackung 50 (auch an verschiedenen vordefinierten Stellen / Positionen) erfolgen kann. So kann es notwendig sein, dass eine zentrierte Anordnung des Prüfgeräts 10 an (genau einer oder an verschiedenen vordefinierten Stellen / Positionen) der Verpackung 50 erfolgen muss, damit eine korrekte Untersuchung durchgeführt werden kann. Hierzu kann der Benutzer das (mobile) Prüfgerät 10 (idealerweise an einem Handgriff) mit Hilfe der Positionierungsstruktur 40 durch Drehung oder Schwenkung (zur Verpackung 50) an vordefinierten Stellen / Positionen bewegen, um dort einzelne Messergebnisse zu erfassen. Die Erfassungsanordnung 30 weist eine Sendeeinheit 31, welche als eine Laserquelle ausgeführt ist, zur Aussendung einer Messeinwirkung 38, in Form einer Emittierung eines Laserstrahls 38, auf. Die Positionierungsstruktur 40 kann dabei eine derartige geometrische Ausbildung und/oder geometrische Anordnung aufweisen, dass die Sendeeinheit 31 auf einen Anordnungsbereich A (Messbereich) ausgerichtet wird, wenn das Prüfgerät 10 an die Verpackung 50 angebracht wird. Dadurch kann bewirkt werden, dass der Laserstrahl 38, durch die (zumindest teilweise transparente) Verpackung 50 und durch das Lebensmittel 50.4, insbesondere (ggf. ausschließlich) durch die Gasphase G des Lebensmittels 50.4, hindurchtritt. Bspw. trifft die Messeinwirkung 38 zunächst auf eine erste Stelle der Wandung der Verpackung 50, dann auf die Gasphase G und tritt anschließend durch eine zweite Stelle der Wandung der Verpackung 50 wieder aus. Hierbei kann durch das Lebensmittel 50.4, also insbesondere durch die Gasphase G, eine Veränderung der Messeinwirkung 38 verursacht werden. Diese Veränderung ist in einem Spektrum des Laserstrahls 38 identifizierbar. Daher kann eine Empfangseinheit 32 genutzt werden, um den Laserstrahl 38 durch eine Photodiode der Empfangseinheit 32 als Lichtdetektor 32 zu erfassen. Insbesondere wird hierbei ausschließlich eine Intensität des Lichts 38 erfasst. Wenn gemäß einem weiteren Vorteil während dieses Empfangs ein Parameter, wie eine Wellenlänge, der Sendeeinheit 31 variiert wird, kann hierdurch ein Spektrum ermittelt werden.

Nun kann es möglich sein, dass durch eine Auswerteeinheit 14 und/oder durch ein externes Gerät, wie ein mobiles Kommunikationsgerät 1 und/oder durch eine Datenverarbeitungsanlage 100, das erfasste Spektrum analysiert wird. Insbesondere kann das Spektrum einen Peak aufweisen, wobei eine Halbwertsbreite dieses Peaks für einen Gesamtdruck des Lebensmittels 50.4 spezifisch ist. In anderen Worten kann anhand der Erfassung der Empfangseinheit 32 der Druck als ein Parameter des Lebensmittels 50.4 erfasst werden. Um nun eine Eigenschaft des Lebensmittels 50.4, wie einen CO₂-Gehalt, zu bestimmen, muss ggf. noch ein weiterer Parameter erfasst werden. Zu diesem Zweck kann die Erfassungsanordnung 30 weiter einen Temperatursensor 70 aufweisen, welcher bspw. als optischer Sensor, insbesondere mit einem Laser, z. B. als Bolometer oder Pyrometer, ausgebildet ist. Dieser ist ebenfalls derart an der Positionierungsstruktur 40 befestigt, dass bei der Anordnung des Prüfgeräts 10 an der Verpackung 50 eine Ausrichtung des Temperatursensors 70 in Richtung der Verpackung 50, vorzugsweise der Flüssigkeit F, erfolgt. Entsprechend kann der Temperatursensor 70 in definierter Weise tiefer bzw. unterhalb der Empfangseinheit 32 und/oder der Sendeeinheit 31 angeordnet sein, wobei sich die Richtungsangaben vorteilhafterweise auf eine Richtung der Schwerkraft beziehen, welche auf die Flüssigkeit F wirkt. Entsprechend sollte zur Untersuchung die Verpackung 50 mit dem Lebensmittel 50.4 möglichst derart positioniert werden, dass sich ein horizontaler Füllstand 50.5 (orthogonal zur Richtung der Schwerkraft) einstellt.

Von Vorteil ist es außerdem, wenn das Prüfgerät 10 eine Halteeinheit 41 aufweist. Die Halteeinheit 41 kann zur formschlüssigen Befestigung des Prüfgeräts 10 an der Verpackung 50 dienen, z. B. an einem Verschluss 50.1 der Verpackung 50. Hierzu ist es denkbar, dass die Halteeinheit 41 eine konische Aufnahme aufweist, um den Verschluss 50.1 und/oder einen Flaschenhals 50.6 aufzunehmen. Dies ermöglicht eine Zentrierung des Prüfgeräts 10 an der Verpackung 50, insbesondere an der Flasche 50.2 in Bezug auf den Flaschenhals 50.6. Dies ist erforderlich, um eine exakte Positionierung der Sendeeinheit 31 und/oder der Empfangseinheit 32 und/oder des Temperatursensors 70 mit einem vorbestimmten Abstand und/oder Winkel an der Verpackung 50 zu ermöglichen.

Optional kann es möglich sein, dass die Halteeinheit 41 einen Dreh- und/oder Schwenkmechanismus 42 bereitstellt. Bspw. ist die Halteeinheit 41 als ein sogenannter Verschlussgreifer (Chuck) ausgebildet, sodass ein Drehmomentmesssystem 43 bereitgestellt wird, um bei einer Drehung an einem Verschluss 50.1 der Verpackung 50 ein Drehmoment zu erfassen. Dies hat den Vorteil, dass zur weitergehenden Prüfung des Lebensmittels 50.4 ein Drehmoment durch ein Öffnen und/oder Schließen der Flasche 50.2 gemessenen werden kann, welches ebenfalls einen Rückschluss auf den Gasgehalt liefert. Ferner kann es möglich sein, dass das Prüfgerät zur Bedienung eine Eingabevorrichtung 85 aufweist, welche z. B. einen Taster zum Einschalten und/oder einen (weiteren) Auswahltaster zur Bedienung aufweist.

Durch die Nutzung eines Temperatursensors 70 zur Erfassung der Temperatur des Lebensmittels 50.4 und durch eine Auswertung der erfassten Temperatur und des erfassten Drucks kann in vorteilhafter Weise eine Eigenschaft des Lebensmittels 50.4, bevorzugt ein CO₂-Gehalt des Lebensmittels 50.4, bestimmt werden. Somit kann das Prüfgerät 10 sämtliche Sensoren bereitstellen, um die Rohdaten zur Bestimmung dieser Eigenschaft zu erfassen. Es kann dabei möglich sein, dass die Auswertung zur Bestimmung der Eigenschaft durch das Prüfgerät 10 selbst oder alternativ durch ein externes Gerät 100, 1 anhand der Rohdaten erfolgt. Insbesondere kann es möglich sein, dass Kalibrierungsdaten notwendig sind, um anhand der Rohdaten die Eigenschaft zu bestimmen.

Bspw. kann eine Kommunikationsvorrichtung 20 bei dem Prüfgerät 10 vorgesehen sein, um die Rohdaten an ein externes Gerät, wie ein mobiles Kommunikationsgerät 1 und/oder eine Datenverarbeitungsanlage 100, wie einen Server, zu übertragen. Entsprechend kann dann die Auswertung zur Bestimmung der Eigenschaft durch das externe Gerät erfolgen. Dabei kann es auch möglich sein, dass das mobile Kommunikationsgerät 1 als Vermittler zur Datenverarbeitungsanlage 100 dient. In diesem Fall können die Rohdaten (also z. B. ein Erfassungsergebnis der Erfassung der Erfassungsanordnung 30) zunächst von der Kommunikationsvorrichtung 20 an das mobile Kommunikationsgerät 1 und anschließend von dem mobilen Kommunikationsgerät 1 an die Datenverarbeitungsanlage 100 über eine Kommunikation 5, z. B. über ein Netzwerk, insbesondere ein Mobilfunknetz und/oder ein Internet, übertragen werden.

Die Datenverarbeitungsanlage 100 und/oder das mobile Kommunikationsgerät 1 umfasst z. B. eine Datenbank 110, in welcher Kalibrierungsdaten gespeichert sind. Diese können genutzt werden, um die Erfassung der Erfassungsanordnung 30 zu parametrisieren und/oder um das Erfassungsergebnis (also die Rohdaten) der Erfassung auszuwerten. Bspw. muss die Erfassung und/oder die Auswertung für unterschiedliche Verpackungsgeometrien und/oder für unterschiedliche Lebensmittel in unterschiedlicher Weise erfolgen. Dies wird durch die Kalibrierungsdaten berücksichtigt, welche hierzu bspw. empirisch oder anhand von Labormessungen ermittelt worden sind.

Zur Auswahl der passenden Kalibrierungsdaten kann es von Vorteil sein, wenn diese manuell (z. B. durch eine Eingabevorrichtung 85) und/oder automatisiert ausgewählt werden. Zur automatisierten Auswahl kann z. B. eine Bildinformation über das Lebensmittel 50.4 und/oder die Verpackung 50 genutzt werden, welche ggf. durch das mobile Kommunikationsgerät 1 erfasst und/oder dem Prüfgerät 10 und/oder der Datenverarbeitungsanlage 100 zur Verfügung gestellt wird. Bspw. kann durch ein Sensorelement 2, wie eine Kamera des Kommunikationsgeräts 1, ein Bild der Verpackung 50 mit dem Lebensmittel 50.4 aufgenommen und ggf. an das Prüfgerät 10 übertragen werden.

Anschließend kann ein Ergebnis der Untersuchung, z. B. ein Ergebnis der Auswertung, durch eine Anzeigevorrichtung 80 des Prüfgeräts 10 und/oder durch ein Anzeigemittel 3 des mobilen Kommunikationsgeräts 1 ausgegeben werden. Es erfolgt bspw. eine direkte Ausgabe der Messwerte, also Werte über die Höhe und/oder Art der gemessenen Eigenschaft des Lebensmittels 50.4, wie eine Höhe des Gasgehalts. Auch kann die Ausgabe vereinfacht, z. B. gemäß einer Ampeldarstellung (rot-gelb-grün) dreistufig, erfolgen. Hierzu können als Anzeigemittel 3 bzw. als Anzeigevorrichtung 80 auch mehrfarbige Leuchtdioden genutzt werden.

Ein besonderer Vorteil ergibt sich daraus, dass die Untersuchung mobil durchgeführt werden kann. Hierzu ist das Prüfgerät 10 als Handheld-Gerät ausgeführt. Um den mobilen Betrieb möglich zu machen, kann ein Energiespeicher 60 zur netzunabhängigen mobilen Energieversorgung vorgesehen sein. Dieser ist bspw. als Akkumulator ausgebildet und in einem Gehäuse des Prüfgeräts 10 aufgenommen.

Die voranstehende Erläuterung der Ausführungsformen beschreibt die vorliegende Erfindung ausschließlich im Rahmen von Beispielen. Selbstverständlich können einzelne Merkmale der Ausführungsformen, sofern technisch sinnvoll, frei miteinander kombiniert werden, ohne den Rahmen der vorliegenden Erfindung, definiert durch die Ansprüche, zu verlassen.

### Bezugszeichenliste

- 1: Kommunikationsgerät
- 2: Sensorelement
- 3: Anzeigemittel
- 5: Kommunikation

- 10: Prüfgerät
- 14: Auswerteeinheit

- 20: Kommunikationsvorrichtung

- 30: Erfassungsanordnung, Messanordnung
- 31: Sendeeinheit, Lichtquelle, Laserquelle
- 32: Empfangseinheit, Lichtdetektor, optischer Sensor
- 38: Laserstrahl, Messeinwirkung

- 40: Positionierungsstruktur
- 41: Halteeinheit, Positionierungseinheit
- 42: Dreh- und/oder Schwenkmechanismus
- 43: Drehmomentmesssystem

- 50: Verpackung
- 50.1: Deckel
- 50.2: Flasche, Flüssigkeitsbehälter
- 50.3: Kopfraum
- 50.4: Lebensmittel, insbesondere Getränk
- 50.5: Füllstand
- 50.6: Flaschenhals

- 60: Energiespeicher, Energieversorgung
- 70: Temperatursensor

- 80: Anzeigevorrichtung
- 85: Eingabevorrichtung

- 100: Datenverarbeitungsanlage, Server
- 110: Datenbank

- A: Anordnungsbereich
- F: Flüssigkeit
- G: Gasphase

## Patentansprüche

1. Prüfgerät (10) zur zerstörungsfreien Erfassung der Gas-Konzentration bei Lebensmitteln (50.4) in Verpackungen (50), aufweisend:
- eine Erfassungsanordnung (30) zur Erfassung wenigstens eines Parameters bei einem Lebensmittel (50.4) in einer Verpackung (50), wobei der Parameter für eine zu untersuchende Eigenschaft des Lebensmittels (50.4) spezifisch ist,
wobei die Erfassungsanordnung (30) eine Sendeeinheit (31) zur Aussendung einer Messeinwirkung (38) und eine Empfangseinheit (32) zur Erfassung der ausgesendeten Messeinwirkung (38) aufweist, wobei die Sendeeinheit (31) als eine Laserquelle (31) und die Empfangseinheit (32) als ein elektromagnetischer Sensor (32) und die Messeinwirkung (38) als eine Laserstrahlung ausgeführt ist, um eine Veränderung eines Spektrums der Strahlung zu erfassen, welche für den Parameter spezifisch ist,
wobei das Prüfgerät (10) als ein Handheld-Gerät ausgebildet ist,
wobei eine Positionierungsstruktur (40) vorgesehen ist, um eine äußere Positionierung der Erfassungsanordnung (30) an der Verpackung (50) durchzuführen.

2. Prüfgerät (10) nach Anspruch 1,
**dadurch gekennzeichnet,**
**dass** die zu untersuchende Eigenschaft ein Gasgehalt des Lebensmittels (50.4) in der Verpackung (50) ist,
und/oder dass der Parameter ein Druck und/oder eine Temperatur bei dem Lebensmittel (50.4) ist.

3. Prüfgerät (10) nach einem der vorhergehenden Ansprüche,
**dadurch gekennzeichnet,**
**dass** eine Kommunikationsvorrichtung (20) vorgesehen ist, um eine, z.B. drahtlose, Datenkommunikation mit einem mobilen Kommunikationsgerät (1) und/oder einer zentralen Datenverarbeitungsanlage (100) durchzuführen, um ein Ergebnis der Erfassung über die Datenkommunikation zu übertragen.

4. Prüfgerät (10) nach einem der vorhergehenden Ansprüche,
**dadurch gekennzeichnet,**
**dass** eine Positionierungsstruktur (40) vorgesehen ist, um eine äußere Positionierung der Erfassungsanordnung (30) an der Verpackung (50) gemäß einer vorbestimmten Anordnung durchzuführen,, insbesondere mit vorbestimmten Abständen und/oder zentriert zur Verpackung (50),
und/oder dass eine Positionierungsstruktur (40) zur zumindest teilweisen Anordnung der Verpackung (50) mit dem Lebensmittel (50.4) in einen Anordnungsbereich (A) als Messbereich (A) vorgesehen ist, wobei eine Sendeeinheit (31) und eine Empfangseinheit (32) der Erfassungsanordnung (30) durch die Positionierungsstruktur (40) auf den Messbereich ausgerichtet sind.

5. Prüfgerät (10) nach einem der vorhergehenden Ansprüche,
**dadurch gekennzeichnet,**
**dass** ebenfalls eine Halteeinheit (41), vorzugsweise Positionierungseinheit (41), möglich ist, um das Prüfgerät (10) an der Verpackung (50) lösbar zu befestigen,
und/oder dass eine Halteeinheit (41) möglich ist, welche als Verschlussgreifer zur Drehmomentmessung ausgebildet ist, um an einem Flaschenverschluss (50.3) der als Flasche (50.2) ausgebildeten Verpackung (50) fixiert zu werden,
und/oder dass die Möglichkeit besteht, dass eine Halteeinheit (41) zur Adaption an einen Kopfraum (50.3) einer als Flasche (50.2) ausgebildeten Verpackung (50) ausgebildet ist, vorzugsweise um das Prüfgerät (10) konzentrisch an einem Flaschenverschluss (50.3) zu befestigen,
und/oder dass eine Halteeinheit (41) vorsehbar und als Zentriereinheit ausgebildet ist, sodass eine Zentrierung des Prüfgeräts (10) an der Verpackung (50) in einer Messposition durch die Halteeinheit (41) erfolgt.

6. Prüfgerät (10) nach einem der vorhergehenden Ansprüche,
**dadurch gekennzeichnet,**
**dass** die Sendeeinheit (31) derart auf die Empfangseinheit (32) und einen Anordnungsbereich (A) ausgerichtet ist, dass eine Messeinwirkung (38) zunächst in den Anordnungsbereich (A) und dann zur Empfangseinheit (32) gelangt, um eine Veränderung der Messeinwirkung (38) durch das Lebensmittel (50.4) zu erfassen, und/oder dass eine Anzeigevorrichtung (80) zur Darstellung eines mehrstufigen Untersuchungsergebnisses vorgesehen ist,
und/oder dass für einen portablen Betrieb des Prüfgeräts (10) ein Energiespeicher (60), vorzugsweise ein Akkumulator, zur mobilen Energieversorgung vorgesehen ist, und/oder dass die Erfassungsanordnung (30) einen optischen Temperatursensor (70), insbesondere ein Bolometer oder Pyrometer, aufweist, um eine Temperatur bei dem Lebensmittel (50.4) in der Verpackung (50) zu erfassen.

7. System zur Untersuchung, insbesondere zur zerstörungsfreien Druck- und Temperaturerfassung, zur Erfassung von Gasgehalten, bei Lebensmitteln (50.4) in Verpackungen (50), aufweisend:
- ein Prüfgerät (10) zur Erfassung wenigstens eines Parameters bei einem Lebensmittel (50.4) in einer Verpackung (50), wobei der Parameter für eine zu untersuchende Eigenschaft des Lebensmittels (50.4) spezifisch ist,
- eine Auswerteeinheit (14) zur Auswertung der Erfassung, um die zu untersuchende Eigenschaft des Lebensmittels (50.4) zu bestimmen,
wobei das Prüfgerät (10) als ein Handheld-Gerät nach einem der vorhergehenden Ansprüche ausgebildet ist.

8. System nach einem der vorhergehenden Ansprüche,
**dadurch gekennzeichnet,**
**dass** die Auswerteeinheit (14) Teil des Prüfgeräts (10) ist, sodass die Auswertung durch das Prüfgerät (10) durchgeführt wird,
und/oder dass die optionale Nutzung eines mobilen Kommunikationsgerät (1) vorgesehen ist, welches die Auswerteeinheit (14) umfasst, um die Auswertung separat vom Prüfgerät (10) durchzuführen,
und/oder dass optional ein mobiles Kommunikationsgerät (1) vorgesehen ist, welches wenigstens ein Sensorelement (2) aufweist, um eine Information über das Lebensmittel (50.4) in der Verpackung (50) separat vom Prüfgerät (10) zu erfassen, sodass die Untersuchung, insbesondere die Bestimmung der zu untersuchenden Eigenschaft des Lebensmittels (50.4), in Abhängigkeit von der erfassten Information und des erfassten Parameters erfolgt.

9. Verfahren zur Untersuchung, insbesondere zur zerstörungsfreien Druck- und Temperaturerfassung, vorzugsweise zur Erfassung eines Gasgehaltes, bei Lebensmitteln (50.4) in Verpackungen (50),
**dadurch gekennzeichnet,**
**dass** die nachfolgenden Schritte durchgeführt werden:
a) Mobiles Erfassen von wenigstens einem Parameter bei einem Lebensmittel (50.4) in einer Verpackung (50) durch ein tragbares Prüfgerät (10), wobei der Parameter für eine zu untersuchende Eigenschaft des Lebensmittels (50.4) spezifisch ist,
b) Auswerten zumindest der Erfassung des wenigstens einen Parameters, um ein Untersuchungsergebnis zu bestimmen,
c) Ausgeben des Untersuchungsergebnisses,
wobei ein Prüfgerät (10) nach einem der vorhergehenden Ansprüche und/oder ein System nach einem der vorhergehenden Ansprüche betrieben wird.

10. Verfahren nach einem der vorhergehenden Ansprüche,
**dadurch gekennzeichnet,**
**dass** vor Schritt a) eine Information, vorzugsweise Bildinformation, durch ein optisches Sensorelement (2), vorzugsweise eines mobilen Kommunikationsgeräts (1), insbesondere eines Mobilfunkkommunikationsgeräts, über die Verpackung (50) und/oder das Lebensmittel (50.4) in der Verpackung (50) erfasst werden kann, und bei Schritt b) das Auswerten auch anhand dieser Information erfolgen kann, bevorzugt um die Art der Verpackung (50) und/oder des Lebensmittels (50.4) zu berücksichtigen,
und/oder dass vor Schritt a) wenigstens eine Information über die Verpackung (50) und/oder über das Lebensmittel (50.4) durch eine Kommunikation des Prüfgeräts (10) oder eines mobilen Kommunikationsgeräts (1) an eine externe Datenverarbeitungsanlage (100) übermittelt wird, und anhand einer Datenbank (110) der Datenverarbeitungsanlage (100) wenigstens eine Kalibrierungsinformation in Abhängigkeit von der übermittelten Information ermittelt wird, um anhand der Kalibrierungsinformation die Erfassung gemäß Schritt a) und/oder die Auswertung gemäß Schritt b) durchzuführen,
und/oder dass Schritt a) und/oder b) und/oder c) autonom durch das Prüfgerät (10) durchgeführt wird,
und/oder dass die Schritte b) und/oder c) zumindest teilweise durch ein externes Gerät, vorzugsweise durch die Datenverarbeitungsanlage (100) und/oder durch das mobile Kommunikationsgerät (1), durchgeführt werden, wobei vorzugsweise hierzu eine Datenkommunikation des Prüfgeräts (10) über ein Datennetzwerk mit dem externen Gerät erfolgt.

11. Verfahren nach einem der vorhergehenden Ansprüche,
**dadurch gekennzeichnet,**
**dass** das Erfassen gemäß Schritt a) und/oder das Auswerten gemäß Schritt b) eine spektroskopische Untersuchung elektromagnetischer Wellen umfasst.

12. Verfahren nach einem der vorhergehenden Ansprüche,
**dadurch gekennzeichnet,**
**dass** vor Schritt b) wenigstens eine geometrische Eigenschaft der Verpackung (50) messtechnisch erfasst werden kann, vorzugsweise durch ein mobiles Kommunikationsgerät (1), bevorzugt durch eine Bildaufnahme, und bei Schritt b) ausgewertet werden kann.

13. Verfahren nach einem der vorhergehenden Ansprüche,
**dadurch gekennzeichnet,**
**dass** gemäß Schritt a) zumindest die Temperatur und/oder der Druck gemessen wird, um einen Gasgehalt in der Verpackung (50) aus den Messwerten dieser Messung zu bestimmen, vorzugsweise in Abhängigkeit von wenigstens einer Kalibrierungsinformation, welche in Abhängigkeit von dem Lebensmittel (50.4) in der Verpackung (50) ermittelt werden.

## Claims

1. Testing device (10) for non-destructive detection of the gas concentration in foodstuffs (50.4) in packages (50), comprising:
- a detection arrangement (30) for detecting at least one parameter in a foodstuff (50.4) in a package (50), the parameter being specific to a property of the foodstuff (50.4) to be examined,
wherein the detection arrangement (30) comprises a transmitting unit (31) for transmitting a measuring effect (38) and a receiving unit (32) for detecting the transmitted measuring effect (38), wherein the transmitting unit (31) is designed as a laser source (31) and the receiving unit (32) is designed as an electromagnetic sensor (32) and the measuring effect (38) is designed as a laser radiation in order to detect a change in a spectrum of the radiation which is specific to the parameter,
wherein the testing device (10) is designed as a handheld device,
wherein a positioning structure (40) is provided to perform an external positioning of the detection arrangement (30) on the package (50).

2. Testing device (10) according to claim 1,
**characterized in that**
the property to be investigated is a gas content of the foodstuff (50.4) in the package (50), and/or that the parameter is a pressure and/or a temperature at the foodstuff (50.4).

3. Testing device (10) according to any one of the preceding claims,
**characterized in that**
A communication device (20) is provided to perform a data communication, e.g. wireless, with a mobile communication device (1) and/or a central data processing system (100) to transmit a result of the detection via the data communication.

4. Testing device (10) according to any one of the preceding claims,
**characterized in that**
a positioning structure (40) is provided to perform an external positioning of the detection arrangement (30) on the package (50) according to a predetermined arrangement, in particular with predetermined distances and/or centered to the package (50),
and/or **in that** a positioning structure (40) is provided for at least partially arranging the packaging (50) with the foodstuff (50.4) in an arrangement area (A) as a measuring area (A), a transmitting unit (31) and a receiving unit (32) of the detection arrangement (30) being aligned with the measuring area by the positioning structure (40).

5. Testing device (10) according to any one of the preceding claims,
**characterized in that**
a holding unit (41), preferably a positioning unit (41), is also possible in order to detachably fasten the testing device (10) to the packaging (50),
and/or **in that** a holding unit (41) is possible, which is designed as a closure gripper for torque measurement, in order to be fixed to a bottle closure (50.3) of the packaging (50) designed as a bottle (50.2),
and/or **in that** it is possible for a holding unit (41) to be designed for adaptation to a head space (50.3) of a package (50) designed as a bottle (50.2), preferably in order to fasten the testing device (10) concentrically to a bottle closure (50.3),
and/or **in that** a holding unit (41) can be provided and is designed as a centering unit, so that centering of the testing device (10) on the packaging (50) takes place in a measuring position by means of the holding unit (41).

6. Testing device (10) according to any one of the preceding claims,
**characterized in that**
the transmitting unit (31) is aligned with the receiving unit (32) and an arrangement area (A) in such a way that a measuring effect (38) first reaches the arrangement area (A) and then the receiving unit (32) in order to detect a change in the measuring effect (38) by the foodstuff (50.4),
and/or that a display device (80) is provided for displaying a multi-stage examination result, and/or **in that** an energy storage device (60), preferably a rechargeable battery, is provided for portable operation of the testing device (10) for mobile energy supply,
and/or that the detection arrangement (30) comprises an optical temperature sensor (70), in particular a bolometer or pyrometer, to detect a temperature at the foodstuff (50.4) in the packaging (50).

7. System for testing, in particular for non-destructive pressure and temperature detection, for detecting gas contents, in foodstuffs (50.4) in packages (50), comprising:
- a testing device (10) for detecting at least one parameter in a foodstuff (50.4) in a package (50), the parameter being specific to a property of the foodstuff (50.4) to be tested,
- an evaluation unit (14) for evaluating the detection in order to determine the property of the foodstuff (50.4) to be examined,
wherein the testing device (10) is configured as a handheld device according to any one of the preceding claims.

8. A system according to any one of the preceding claims,
**characterized in that**
the evaluation unit (14) is part of the testing device (10), so that the evaluation is carried out by the testing device (10),
and/or that the optional use of a mobile communication device (1) is provided, which comprises the evaluation unit (14) in order to carry out the evaluation separately from the testing device (10),
and/or **in that** optionally a mobile communication device (1) is provided which has at least one sensor element (2) in order to detect information about the foodstuff (50.4) in the packaging (50) separately from the testing device (10), so that the examination, in particular the determination of the property of the foodstuff (50.4) to be examined, is carried out as a function of the detected information and the detected parameter.

9. Method for the examination, in particular for the non-destructive detection of pressure and temperature, preferably for the detection of a gas content, of a foodstuff (50.4) in packages (50),
**characterized in that**
the following steps are carried out:
a) mobile detection of at least one parameter in a foodstuff (50.4) in a package (50) by a portable testing device (10), wherein the parameter is specific to a property of the foodstuff (50.4) to be tested,
(b) evaluating at least the acquisition of the at least one parameter to determine an examination result,
c) issuing the result of the examination,
wherein a testing device (10) according to any one of the preceding claims and/or a system according to any one of the preceding claims is operated.

10. The method according to any one of the preceding claims,
**characterized in that**
before step a), information, preferably image information, can be recorded by an optical sensor element (2), preferably of a mobile communication device (1), in particular a mobile communication device, about the packaging (50) and/or the foodstuff (50.4) in the packaging (50), and in step b), the evaluation can also be carried out on the basis of this information, preferably in order to take into account the type of packaging (50) and/or foodstuff (50.4),
and/or **in that**, before step a), at least one item of information about the packaging (50) and/or about the foodstuff (50.4) is transmitted to an external data processing system (100) by means of a communication of the testing device (10) or of a mobile communication device (1), and at least one item of calibration information is determined on the basis of a database (110) of the data processing system (100) as a function of the transmitted information, in order to carry out the detection according to step a) and/or the evaluation according to step b) on the basis of the calibration information,
and/or that step a) and/or b) and/or c) is carried out autonomously by the testing device (10),
and/or **in that** steps b) and/or c) are carried out at least in part by an external device, preferably by the data processing system (100) and/or by the mobile communication device (1), data communication of the testing device (10) with the external device preferably taking place via a data network for this purpose.

11. The method according to any one of the preceding claims,
**characterized in that**
the detection according to step a) and/or the evaluation according to step b) comprises a spectroscopic examination of electromagnetic waves.

12. The method according to any one of the preceding claims,
**characterized in that**
before step b), at least one geometric property of the packaging (50) can be measured, preferably by a mobile communication device (1), preferably by an image recording, and can be evaluated in step b).

13. The method according to any one of the preceding claims,
**characterized in that**
according to step a) at least the temperature and/or the pressure is measured in order to determine a gas content in the packaging (50) from the measured values of this measurement, preferably in dependence on at least one calibration information which is determined in dependence on the foodstuff (50.4) in the packaging (50).

## Revendications

1. Appareil de contrôle (10) pour la détection non destructive de la concentration de gaz dans des produits alimentaires (50.4) dans des emballages (50), présentant :
- un dispositif de détection (30) pour détecter au moins un paramètre dans un produit alimentaire (50.4) dans un emballage (50), le paramètre étant spécifique à une propriété du produit alimentaire (50.4) à examiner,
le dispositif de détection (30) présentant une unité d'émission (31) pour l'émission d'une action de mesure (38) et une unité de réception (32) pour la détection de l'action de mesure (38) émise, l'unité d'émission (31) étant réalisée sous la forme d'une source laser (31) et l'unité de réception (32) sous la forme d'un capteur électromagnétique (32) et l'action de mesure (38) sous la forme d'un rayonnement laser, afin de détecter une modification d'un spectre du rayonnement qui est spécifique au paramètre,
l'appareil de contrôle (10) étant conçu comme un appareil portatif,
dans lequel une structure de positionnement (40) est prévue pour effectuer un positionnement externe du dispositif de détection (30) sur l'emballage (50).

2. Appareil de contrôle (10) selon la revendication 1,
**caractérisé en ce que**
la propriété à examiner est une teneur en gaz du produit alimentaire (50.4) dans l'emballage (50),
et/ou **en ce que** le paramètre est une pression et/ou une température au niveau du produit alimentaire (50.4).

3. Appareil de contrôle (10) selon l'une des revendications précédentes,
**caractérisé en ce qu'**
un dispositif de communication (20) est prévu pour effectuer une communication de données, par exemple sans fil, avec un appareil de communication mobile (1) et/ou une installation centrale de traitement de données (100), afin de transmettre un résultat de la saisie via la communication de données.

4. Appareil de contrôle (10) selon l'une des revendications précédentes,
**caractérisé en ce que**
une structure de positionnement (40) est prévue pour effectuer un positionnement externe de l'ensemble de détection (30) sur l'emballage (50) selon un agencement prédéterminé, notamment à des distances prédéterminées et/ou centré sur l'emballage (50),
et/ou **en ce qu'**il est prévu une structure de positionnement (40) pour disposer au moins partiellement l'emballage (50) avec le produit alimentaire (50.4) dans une zone de disposition (A) comme zone de mesure (A), une unité d'émission (31) et une unité de réception (32) du dispositif de détection (30) étant orientées sur la zone de mesure par la structure de positionnement (40).

5. Appareil de contrôle (10) selon l'une des revendications précédentes,
**caractérisé en ce que**
une unité de maintien (41), de préférence une unité de positionnement (41), est également possible pour fixer de manière amovible l'appareil de contrôle (10) à l'emballage (50), et/ou **en ce qu'**il est possible d'avoir une unité de maintien (41) qui est conçue comme une pince de fermeture pour la mesure du couple, afin d'être fixée sur une fermeture de bouteille (50.3) de l'emballage (50) conçu comme une bouteille (50.2),
et/ou **en ce qu'**il est possible qu'une unité de maintien (41) soit conçue pour s'adapter à un espace de tête (50.3) d'un emballage (50) conçu comme une bouteille (50.2), de préférence pour fixer l'appareil de contrôle (10) de manière concentrique à une fermeture de bouteille (50.3),
et/ou **en ce qu'**une unité de maintien (41) peut être prévue et est conçue comme une unité de centrage, de sorte qu'un centrage de l'appareil de contrôle (10) sur l'emballage (50) dans une position de mesure est effectué par l'unité de maintien (41).

6. Appareil de contrôle (10) selon l'une des revendications précédentes,
**caractérisé en ce que**
l'unité d'émission (31) est orientée vers l'unité de réception (32) et une zone d'agencement (A) de telle sorte qu'une action de mesure (38) parvient d'abord dans la zone de disposition (A) et ensuite à l'unité de réception (32) afin de détecter une modification de l'action de mesure (38) par l'e produit alimentaire (50.4),
et/ou **en ce qu'**il est prévu un dispositif d'affichage (80) pour représenter un résultat d'examen à plusieurs niveaux,
et/ou **en ce que**, pour un fonctionnement portable de l'appareil de contrôle (10), il est prévu un accumulateur d'énergie (60), de préférence un accumulateur, pour l'alimentation mobile en énergie,
et/ou **en ce que** le dispositif de détection (30) présente un capteur de température optique (70), en particulier un bolomètre ou un pyromètre, afin de détecter une température au niveau de produit alimentaire (50.4) dans l'emballage (50).

7. Système d'analyse, en particulier pour la détection non destructive de la pression et de la température, pour la détection de teneurs en gaz, pour des produits alimentaires (50.4) dans des emballages (50), présentant :
- un appareil de contrôle (10) pour détecter au moins un paramètre dans un produit alimentaire (50.4) dans un emballage (50), le paramètre étant spécifique à une propriété du produit alimentaire (50.4) à examiner,
- une unité d'évaluation (14) pour l'évaluation de la saisie, afin de déterminer la propriété à examiner du produit alimentaire (50.4),
l'appareil de contrôle (10) étant conçu comme un appareil portatif selon l'une des revendications précédentes.

8. Système selon l'une quelconque des revendications précédentes,
**caractérisé en ce que**
l'unité d'évaluation (14) fait partie de l'appareil de contrôle (10), de sorte que l'évaluation est effectuée par l'appareil de contrôle (10),
et/ou **en ce que** l'utilisation optionnelle d'un appareil de communication mobile (1) est prévue, lequel comprend l'unité d'évaluation (14) afin d'effectuer l'évaluation séparément de l'appareil de contrôle (10),
et/ou **en ce qu'**il est prévu en option un appareil de communication mobile (1) qui présente au moins un élément capteur (2) pour saisir une information sur l'e produit alimentaire (50.4) dans l'emballage (50) séparément de l'appareil de contrôle (10), de sorte que l'examen, en particulier la détermination de la propriété à examiner du produit alimentaire (50.4), a lieu en fonction de l'information saisie et du paramètre saisi.

9. Procédé d'analyse, en particulier pour la détection non destructive de la pression et de la température, de préférence pour la détection d'une teneur en gaz, de produits alimentaires (50.4) dans des emballages (50),
**caractérisé en ce que**
les étapes suivantes soient effectuées :
a) détection mobile d'au moins un paramètre dans un produit alimentaire (50.4) dans un emballage (50) par un appareil de contrôle portable (10), le paramètre étant spécifique à une propriété du produit alimentaire (50.4) à examiner,
b) évaluer au moins la détection dudit au moins un paramètre afin de déterminer un résultat d'examen,
c) délivrer le résultat de l'examen,
dans lequel on fait fonctionner un appareil de contrôle (10) selon l'une des revendications précédentes et/ou un système selon l'une des revendications précédentes.

10. Procédé selon l'une quelconque des revendications précédentes,
**caractérisé en ce qu'**
avant l'étape a), une information, de préférence une information d'image, peut être saisie par un élément capteur optique (2), de préférence d'un appareil de communication mobile (1), en particulier d'un appareil de communication mobile, concernant l'emballage (50) et/ou l'aliment (50.4) dans l'emballage (50), et à l'étape b), l'évaluation peut également être effectuée à l'aide de cette information, de préférence pour tenir compte du type d'emballage (50) et/ou du produit alimentaire (50.4),
et/ou **en ce que**, avant l'étape a), au moins une information sur l'emballage (50) et/ou sur le produit alimentaire (50.4) est transmise par une communication de l'appareil de contrôle (10) ou d'un appareil de communication mobile (1) à une installation de traitement de données externe (100), et, à l'aide d'une banque de données (110) de l'installation de traitement de données (100), au moins une information de calibrage est déterminée en fonction de l'information transmise, afin d'effectuer, à l'aide de l'information de calibrage, la saisie selon l'étape a) et/ou l'évaluation selon l'étape b),
et/ou **en ce que** l'étape a) et/ou b) et/ou c) est exécutée de manière autonome par l'appareil de contrôle (10),
et/ou **en ce que** les étapes b) et/ou c) sont exécutées au moins en partie par un appareil externe, de préférence par l'installation de traitement de données (100) et/ou par l'appareil de communication mobile (1), une communication de données de l'appareil de contrôle (10) étant de préférence effectuée à cet effet avec l'appareil externe par l'intermédiaire d'un réseau de données.

11. Procédé selon l'une quelconque des revendications précédentes,
**caractérisé en ce que**
la détection selon l'étape a) et/ou l'évaluation selon l'étape b) comprend un examen spectroscopique d'ondes électromagnétiques.

12. Procédé selon l'une quelconque des revendications précédentes,
**caractérisé en ce qu'**
avant l'étape b), au moins une propriété géométrique de l'emballage (50) peut être saisie par une technique de mesure, de préférence par un appareil de communication mobile (1), de préférence par une prise de vue, et peut être évaluée lors de l'étape b).

13. Procédé selon l'une quelconque des revendications précédentes,
**caractérisé en ce que**
selon l'étape a), on mesure au moins la température et/ou la pression pour déterminer une teneur en gaz dans l'emballage (50) à partir des valeurs de mesure de cette mesure, de préférence en fonction d'au moins une information de calibrage qui est déterminée en fonction du produit alimentaire (50.4) dans l'emballage (50).
